# EUROPEAN PATENT SPECIFICATION

(11) **EP 4 074 331 B1**
(45) Date of publication and mention of the grant of the patent: **03.09.2025**
(21) Application number: 22169278.3
(22) Date of filing: 08.12.2016
(51) Int. Cl.: A61K 38/47, A61K 38/46, C12N 9/40, C12N 9/42, A61P 43/00

(54) **COMPOSITIONS AND METHODS FOR INTERNALIZING ENZYMES**
ZUSAMMENSETZUNGEN UND VERFAHREN ZUR INTERNALISIERUNG VON ENZYMEN
COMPOSITIONS ET MÉTHODES POUR L'INTERNALISATION D'ENZYMES

(30) Priority: 08.12.2015 US 201562264702 P; 25.08.2016 US 201662379629 P
(43) Date of publication of application: 19.10.2022
(62) Divisional of application: 20197224.7
(73) Proprietor: Regeneron Pharmaceuticals, Inc., Tarrytown, NY 10591 (US)
(72) Inventor: BAIK, Andrew, Tarrytown (US); CYGNAR, Katherine, Tarrytown (US)
(74) Representative: J A Kemp LLP

(56) References cited:
- EP-A1- 3 315 606
- WO-A1-2016/044947
- WO-A2-2006/108052
- US-A1- 2005 142 141
- US-A1- 2010 183 577
- US-A1- 2013 243 775
- US-A1- 2015 322 149

## Description

### FIELD

This application is generally directed to compositions and such compositions for use in methods for treating lysosomal storage diseases. This application is directed specifically to targeted protein complexes that contain replacement enzymes and their use in treating lysosomal storage diseases.

### BACKGROUND

Lysosomal storage diseases are a class of rare diseases that affect the degradation of myriad substrates in the lysosome. Those substrates include sphingolipids, mucopolysaccharides, glycoproteins, glycogen, and oligosaccharides, which can accumulate in the cells of those with disease leading to cell death. Organs affected by lysosomal storage diseases include the central nervous system (CNS), the peripheral nervous system (PNS), lungs, liver, bone, skeletal and cardiac muscle, and the reticuloendothelial system.

Options for the treatment of lysosomal storage diseases include enzyme replacement therapy (ERT), substrate reduction therapy, pharmacological chaperone-mediated therapy, hematopoietic stem cell transplant therapy, and gene therapy. An example of substrate reduction therapy includes the use of Miglustat or Eliglustat to treat Gaucher Type 1. These drugs act by blocking synthase activity, which reduces subsequent substrate production. Hematopoietic stem cell therapy (HSCT), for example, is used to ameliorate and slow-down the negative central nervous system phenotype in patients with some forms of MPS. See R.M. Boustany, "Lysosomal storage diseases--the horizon expands," 9(10) Nat. Rev. Neurol. 583-98, Oct. 2013. Table 1 lists some lysosomal storage diseases and their associated enzymes or other proteins.

**Table 1: Lysosomal Storage Diseases**

| **Class** | **Disease** | **Involved Enzvme/Protein** |
|---|---|---|
| Sphingolipidoses | Fabry disease | α-Galactosidase A |
| | Farber lipogranulomatosis | Ceramidase |
| | Gaucher disease type I | β-Glucosidase |
| | Gaucher disease types II and III | Saposin-C activator |
| | Niemann-Pick diseases types A and B | Sphingomyelinase |
| | GM1-gangliosidosis | β -Galactosidase |
| | GM2-gangliosidosis (Sandhoff) | β -Hexosaminidase A and B |
| | GM2-gangliosidosis (Tay-Sachs) | β -Hexosaminidase A |
| | GM2-gangliosidosis (GM2-activator deficiency) | GM2-activator protein |
| | GM3-gangliosidosis | GM3 synthase |
| | Metachromatic leukodystrophy | Arylsulfatase A |
| | Sphingolipid-activator deficiency | Sphingolipid activator |
| Mucopolysaccharidoses | MPS I (Scheie, Hurler-Scheie, and Hurler disease) | α-Iduronidase |
| | MPS II (Hunter) | Iduronidase-2-sulphatase |
| | MPS IIIA (Sanfilippo A) | Heparan *N*-sulphatase |
| | MPS IIIB (Sanfilippo B) | *N*-acetyl-α-glucosaminidase |
| | MPS IIIC (Sanfilippo C) | Acetyl-CoA; α-glucosamide *N*-acetyltransferase |
| | MPS IIID (Sanfilippo D) | *N*-acetylglucosamine-6-sulphatase |
| | MPS IVA (Morquio syndrome A) | *N*-acetyl galactosamine-6-sulphate sulphatase |
| | MPS IVB (Morquio syndrome B) | β -Galactosidase |
| | MPS VI (Maroteaux-Lamy) | *N*-acetylgalactosamine-4-sulphatase (arylsulphatase B) |
| | MPS VII (Sly disease) | β -Glucuronidase |
| | MPS IX | Hylauronidase |
| Glycogen storage disease | Pompe (glycogen storage disease type II) | α-Glucosidase 2 |
| Lipid metabolism | Lysosomal acid lipase deficiency (LAL-D; Wolman disease) | Lysosomal acid lipase |

Two of the most common LSDs are Pompe disease and Fabry disease. Pompe disease is caused by defective lysosomal enzyme alpha-glucosidase (GAA), which results in the deficient processing of lysosomal glycogen. Accumulation of lysosomal glycogen occurs predominantly in skeletal, cardiac, and hepatic tissues. Infantile onset Pompe causes cardiomegaly, hypotonia, hepatomegaly, and death due to cardiorespiratory failure, usually before 2 years of age. Adult onset Pompe occurs as late as the second to sixth decade and usually involves only skeletal muscle.

Fabry disease is caused by defective lysosomal enzyme alpha-galactosidase A (GLA), which results in the accumulation of globotriaosylceramide within the blood vessels and other tissues and organs. Symptoms associated with Fabry disease include pain from nerve damage and/or small vascular obstruction, renal insufficiency and eventual failure, cardiac complications such as high blood pressure and cardiomyopathy, dermatological symptoms such as formation of angiokeratomas, anhidrosis or hyperhidrosis, and ocular problems such as cornea verticillata, spoke-like cataract, and conjunctival and retinal vascular abnormalities.

Current treatments for lysosomal storage diseases are less than optimal. For example, ERT generally must be administered at a high frequency and a high dose, such as biweekly and up to 40 mg/kg. Also, some replaced enzymes can be immunologically cross-reactive (CRIM), stimulating production of IgG in the subject and thus hindering delivery of the enzyme to the lysosome via the mannose-6-phosphate (M6P) receptor. The IgGs might shield the M6P residues of the replacement enzyme, and the antigen-IgG-antibody complex may be taken up into cellular lysosomes via the Fc receptor, thereby shunting the replacement enzyme preferentially to macrophages.

Delivery of replacement enzymes to the appropriate affected tissues is also inefficient (see Table 2 and Desnick & Schuchman, "Enzyme replacement therapy for lysosomal diseases: lessons from 20 years of experience and remaining challenges," 13 Annu. Rev. Genomics Hum. Genet. 307-35, 2012). For example, patients undergoing long-term enzyme replacement therapy for Infantile Pompe can still suffer from hypernasal speech, residual muscle weakness, ptosis, ostepenia, hearing loss, risk for aspiration, dysphagia, cardiac arrhythmia, and difficulty swallowing. Doses of replacement enzyme oftentimes must be increased over time to 40mg/kg weekly or biweekly.

**Table 2: Inefficient tissue targeting of ERT**

| **Disease** | **Subtype(s)** | **Easy to reach tissue** | **Hard to reach tissue** |
|---|---|---|---|
| Gaucher disease | Type 1 | Spleen, liver, bone marrow | Bone |
| | Types 2 and 3 | Spleen, liver, bone marrow | Bone, brain |
| Fabry disease | Classic and late onset | Vascular endothelium | Kidney, heart |
| Mucopolysaccharidoses | All | Spleen, liver, bone marrow | Bone, brain, cartilage |
| α-Mannosidosis | --- | Spleen, liver, bone marrow | Bone, brain |
| Niemann-Pick disease | Type B | Spleen, liver, bone marrow | Alveolar macrophages |
| Pompe disease | Infantile | --- | Heart, smooth and skeletal muscle |
| | Later onset | --- | Smooth muscle and respiratory skeletal muscle |

Endogenous mannose-6 phosphate receptor (MPR) mediates the transport of most recombinant enzymes to the lysosome. Two complementary forms of MPR exist: cation-independent (CI-MPR), and cation dependent (CD-MPR). Knock-outs of either form have missorted lysosomal enzymes. Lysosomal hydrolases are synthesized in the endoplasmic reticulum and move to the cis-Golgi network, were they are covalently modified by the addition of mannose-6-phosphate (M6P) groups. The formation of this marker depends on the sequential effect of two lysosomal enzymes: UDP-*N*-acetylglucosamine-l-phosphotransferase (G1cNac-phosphotransferase) and *N*-acetylglucosamine-l-phosphodiester-α-*N*-acetyl-glucosaminidase (uncovering enzyme). GlcNac-phosphotransferase catalyzes the transfer of a G1cNAc-1- phosphate residue from UDP-G1cNAc to C6 positions of selected mannoses in high-mannose type oligosaccharides of the hydrolases. Then, the uncovering enzyme removes the terminal G1cNAc, exposing the M6P recognition signal. At the trans-Golgi network, the M6P signal allows the segregation of lysosomal hydrolases from all other types of proteins through selective binding to the M6P receptors. The clathrin-coated vesicles produced bud off from the trans-Golgi network and fuse with late endosomes. At the low pH of the late endosome, the hydrolases dissociate from the M6P receptors and the empty receptors are recycled to the Golgi apparatus for further rounds of transport.

With the exception of β -glucocerebrosidase, which is delivered via the mannose receptor, recombinant lysosomal enzymes comprise M6P glycosylation and are delivered to the lysosome primarily via CI-MPR/IGF2R. Glycosylation/CI-MPR-mediated enzyme replacement delivery however does not reach all clinically relevant tissues (Table 2). Improvement to enzyme replacement therapy have centered on improving CI-MPR delivery by (i) increasing surface expression of CI-MPR using the β2-agonist clenbuterol (Koeberl et al., "Enhanced efficacy of enzyme replacement therapy in Pompe disease through mannose-6-phosphate receptor expression in skeletal muscle," 103(2) Mol. Genet. Metab. 107-12, 2011), (ii) increasing the amount of M6P residues on enzyme (Zhu et al., "Conjugation of mannose-6-phosphate-containing oligosaccharides to acid alpha-glucosidase improves the clearance of glycogen in Pompe mice," 279(48) J. Biol. Chem. 50336-41, 2004), or (iii) fusing an IGF-II domain to the enzyme (Maga et al., "Glycosylation-independent lysosomal targeting of acid alpha-glucosidase enhances muscle glycogen clearance in Pompe mice," 288(3) J. Biol. Chem. 1428-38, 2013). WO 2006/108052 relates to the treatment of lysosomal storage diseases and mentions a lysosomal enzyme coupled to a targeting moiety selected from a receptor binding peptide, an antibody to a receptor or a natural receptor ligand. The targeting through the LDLR-related protein via a natural receptor ligand is mentioned. The use of the anti-transferrin receptor antibody OX26 and other anti-transferrin receptor antibodies is also mentioned.

A large number of lysosomal storage diseases are inadequately treated by enzyme replacement therapy or gene therapy mainly due to poor targeting of the replacement enzyme to the relevant tissue or organ. A need exists for improved enzyme replacement therapies that enhance and promote better tissue biodistribution and lysosomal uptake of the enzyme. Applicants have developed an improved enzyme replacement therapy using CI-MPR independent antibody-guided delivery of enzymes to the lysosome of target affected tissues.

### SUMMARY

The invention is defined in the claims. The present invention provides a composition comprising an enzyme associated with a lysosomal storage disease (LSD) and an antigen-binding protein,
wherein the enzyme is α-glucosidase (GAA),
wherein the antigen-binding protein is an antibody fragment covalently linked to the enzyme,
wherein the antigen-binding protein binds an internalization effector, and
wherein the internalization effector is transferrin receptor.

The present invention also provides a biotherapeutic complex for use in a method of treating a subject suffering from a lysosomal storage disease (LSD), wherein the method comprises administering to the subject the biotherapeutic complex and wherein the biotherapeutic complex enters a lysosome of a cell of the subject and provides an enzyme activity ("replacement enzyme") that replaces the enzymatic activity that is associated with the LSD ("endogenous enzyme"),
wherein the LSD is Pompe disease, and
wherein the biotherapeutic complex comprises:
   (a) the replacement enzyme, wherein the replacement enzyme is α-glucosidase (GAA), and
   (b) an antigen binding protein that binds an internalization effector, wherein the antigen-binding protein is an antibody fragment covalently linked to the replacement enzyme, and wherein the internalization effector is transferrin receptor.

Applicants have discovered that replacement enzymes can be effectively delivered to the lysosome of a specific target cell when associated with a cell surface targeting entity. This enzyme and targeting entity combination is referred to as a biotherapeutic complex. Thus, in one aspect, the disclosure is a composition, *i.e.,* a biotherapeutic complex that comprises an enzyme and an antigen-binding protein. The enzyme is associated with a lysosomal storage disease (LSD) and the antigen-binding protein binds an internalization effector. The internalization effector mediates cell binding and uptake into a lysosome compartment.

In some aspects described herein, the enzyme is any one of α-galactosidase, β- galactosidase, α-glucosidase, β-glucosidase, saposin-C activator, ceramidase, sphingomyelinase, β-hexosaminidase, GM2 activator, GM3 synthase, arylsulfatase, sphingolipid activator, α-iduronidase, iduronidase-2-sulfatase, heparin *N*-sulfatase, *N*-acetyl- α-glucosaminidase, α-glucosamide *N*-acetyltransferase, *N-*acetylglucosamine-6-sulfatase, *N*-acetylgalactosamine-6- sulfate sulfatase, *N*-acetylgalactosamine-4-sulfatase, β-glucuronidase, and hyaluronidase. In some aspects, the enzyme is an isozyme that has an activity the same as or similar to any one or more of those enzymes listed above. In some aspects, a-glucosidase activity can be provided by an isozyme such as sucrase-isomaltase (SI), maltase-glucoamylase (MGAM), glucosidase II (GANAB), or neutral a-glucosidase (C GNAC). In another aspect, α-galactosidase A activity can be provided by an isozyme such as α-*N-*acetylgalactosaminidase that is engineered to gain GLA activity.

In some aspects, the antigen-binding protein is any protein that can bind to one or more internalization effectors. In more specific aspects, the antigen-binding protein is any one or more of a receptor-fusion molecule, a trap molecule, a receptor-Fc fusion molecule, an antibody, an Fab fragment, an F(ab')2 fragment, an Fd fragment, an Fv fragment, a single-chain Fv (scFv) molecule, a dAb fragment, an isolated complementarity determining region (CDR), a CDR3 peptide, a constrained FR3-CDR3-FR4 peptide, a domain-specific antibody, a single domain antibody, a domain-deleted antibody, a chimeric antibody, a CDR-grafted antibody, a diabody, a triabody, a tetrabody, a minibody, a nanobody, a monovalent nanobody, a bivalent nanobody, a small modular immunopharmaceutical (SMIP), a camelid antibody (VHH heavy chain homodimeric antibody), a shark variable IgNAR domain, and the like. In one particular aspect, the antigen-binding protein is a bispecific antibody which binds to the internalization effector and the enzyme.

In some aspects, the internalization effector is a receptor protein, or a ligand that binds a receptor protein, which sits in, on, or at the cell membrane and can be endocytosed. In more specific aspects, the internalization effector is any one or more of CD63, MHC-I, Kremen-1, Kremen-2, LRP5, LRP6, LRP8, transferrin receptor, LDL-receptor, LDL-related protein 1 receptor, ASGR1, ASGR2, amyloid precursor protein-like protein-2 (APLP2), apelin receptor (APLNR), PRLR (prolactin receptor), MAL (Myelin And Lymphocyte protein, a.k.a. VIP17), IGF2R, vacuolar-type H+ ATPase, diphtheria toxin receptor, folate receptor, glutamate receptors, glutathione receptor, leptin receptor, scavenger receptor, SCARA1-5, SCARB 1-3, and CD36. In certain aspects, the internalization effector is a kidney specific internalizer, such as CDH16 (Cadheri-16), CLDN16 (Claudn-16), KL (Klotho), PTH1R (parathyroid hormone receptor), SLC22A13 (Solute carrier family 22 member 13), SLC5A2 (Sodium/glucose cotransporter 2), and UMOD (Uromodulin). In other certain aspects, the internalization effector is a muscle specific internalizer, such as BMPR1A (Bone morphogenetic protein receptor 1A), M-cadherin, CD9, MuSK (muscle-specific kinase), LGR4/GPR48 (G protein-coupled receptor 48), cholinergic receptor (nicotinic) alpha 1, CDH15 (Cadheri-15), ITGA7 (Integrin alpha-7), CACNG1 (L-type calcium channel subunit gamma-1), CACNAlS (L-type calcium channel subunit alpha-15), CACNG6 (L-type calcium channel subunit gamma-6), SCN1B (Sodium channel subunit beta-1), CHRNA1 (ACh receptor subunit alpha), CHRND (ACh receptor subunit delta), LRRC14B (Leucine-rich repeat-containing protein 14B), and POPDC3 (Popeye domain-containing protein 3). In some specific aspects, the internalization effector is ITGA7, CD9, CD63, APLP2, or PRLR. In some specific aspects, the internalization effector is a macrophage-preferential internalizer, including *e.g.*, VSIG4 (CRIG), MSR1 (CD204), and MMR1 (MCR1, CD206).

The biotherapeutic complex can have any one of several formats. In some aspects , the enzyme is covalently linked to the antigen-binding protein. In one particular aspect, the antigen-binding protein comprises a half-antibody (*i.e.,* a single heavy chain and a single light chain), the enzyme is covalently linked to an immunoglobulin Fc-domain, and the Fc-domain that is covalently linked to the enzyme associates with the Fc-domain of the antigen-binding protein. In another particular aspect, the antigen-binding protein is an antibody, and the enzyme is covalently linked to the C-terminus of the heavy chain (or light chain) of that antibody. In yet another particular aspect, the antigen-binding protein is an antibody, and the enzyme is covalently linked to the N-terminus of the heavy chain (or light chain) of that antibody.

In other aspects, the enzyme is not covalently linked to the antigen-binding protein. In one aspect, the antigen-binding protein binds to both the internalization effector and the enzyme. In a specific aspect, the antigen-binding protein is a bispecific antibody that binds the internalization effector and the enzyme.

In some aspects, the enzyme is GAA or an isozyme having GAA activity, and the internalization effector is CD9, ITGA7, CD63, APLP2, or PRLR. In other aspects, the enzyme is GLA or an isozyme having GLA activity, and the internalization effector is CD9, ITGA7, CD63, APLP2, or PRLR.

In another aspect, the disclosure is a method of treating a subject suffering from a lysosomal storage disease (LSD) comprising the step of administering to the subject a biotherapeutic complex (as described above), wherein the biotherapeutic complex enters a lysosome of a cell of the subject and provides an enzyme activity ("replacement enzyme") that replaces the enzymatic activity that is associated with the LSD ("endogenous enzyme"). LSDs include sphingolipidoses, mucopolysaccharidoses, and glycogen storage diseases. More specifically, the treatable LSD is any one or more of the diseases listed in Table 1, and the replacement enzyme has the activity of the corresponding enzyme listed in Table 1. In a specific aspect, the LSD is Pompe disease and the associated enzyme is α-glucosidase (GAA). In another specific aspect, the LSD is Fabry disease and the associated enzyme is α-Galactosidase A (GLA). In yet another specific example, the LSD is lysosomal acid lipase-deficiency (LAL-D) and the associated enzyme is lysosomal acid lipase (LIPA).

In one aspect, the replacement enzyme does not induce an immunological reaction in the subject. In some cases, the replacement enzyme is an isozyme. For example, when the endogenous enzyme is α-glucosidase, the isozyme is a different protein that provides the same or similar enzymatic activity as α-glucosidase, such as sucrase-isomaltase (SI), maltase-glucoamylase (MGAM), glucosidase II (GANAB), and neutral a-glucosidase (C GNAC). When the endogenous enzyme is α-galactosidase A (GLA), the isozyme is a different protein that provides the same or similar enzymatic activity as α-galactosidase A, such as α-*N*-acetylgalactosaminidase that is engineered to gain GLA activity.

In one aspect, the disclosure is a method for selecting or screening a biotherapeutic complex containing an enzyme and an antigen-binding protein that effectively replaces an enzyme in a patient in need thereof. In one aspect, the biotherapeutic complex is administered to a model system and the model system is assessed for replaced enzyme activity. In one aspect, the model system is an animal that lacks expression of the enzyme and expresses an antigen cognate of the antigen-binding protein. In one aspect, the animal model is a mouse that expresses a humanized cognate of the antigen-binding protein and with a knock-out of the gene that encodes the enzyme.

### DRAWINGS

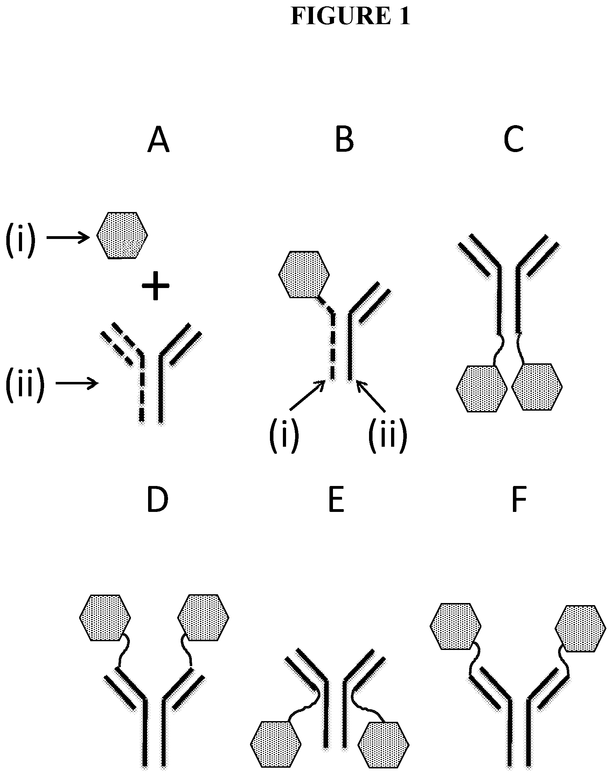

Figure 1 schematically represents biotherapeutic complexes. Panel A depicts a biotherapeutic complex comprising a bispecific antibody (ii) and a replacement enzyme (i). Panel B depicts an enzyme-Fc fusion polypeptide (i) associating with an internalization effector- specific half-body (ii) to form the biotherapeutic complex. Panel C depicts a replacement enzyme (hexagon) covalently linked to the C-terminus of the heavy chain of an anti-internalization effector antibody. Panel D depicts a replacement enzyme (hexagon) covalently linked to the N-terminus of the heavy chain of an anti-internalization effector antibody. Panel E depicts a replacement enzyme (hexagon) covalently linked to the C-terminus of the light chain of an anti-internalization effector antibody. Panel F depicts a replacement enzyme (hexagon) covalently linked to the N-terminus of the light chain of an anti-internalization effector antibody. The curved lines in panels C, D, E and F represent linkers.

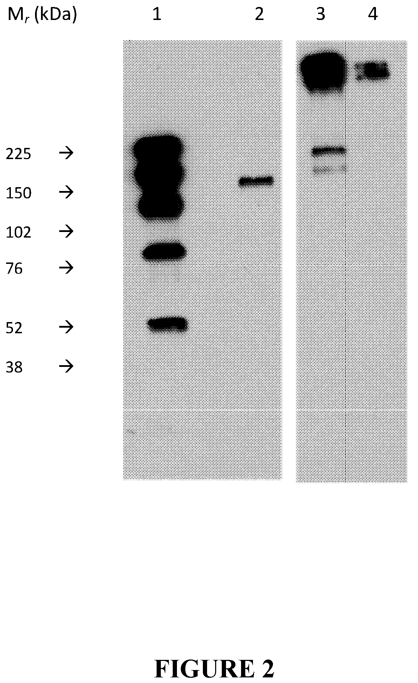

Figure 2 depicts an anti-hFc non-reduced western blot of CHO cell supernatants expressing internalization effector binding protein (IE-BP) or lysosomal storage disease replacement protein (LSD-RP). Lane 1 was loaded with anti-CD63 IgG4, lane 2 with GAA-Fc knob, lane 3 with anti-CD63 IgG4 GAA, and lane 4 with GAA anti-CD63 IgG4.

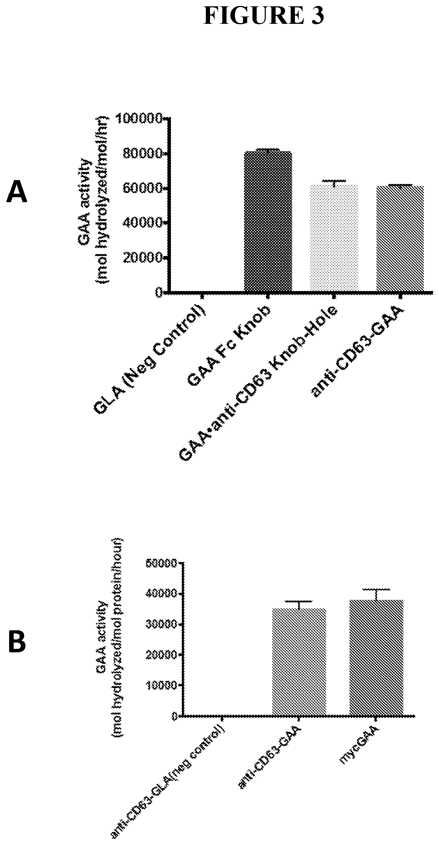

Figure 3 depicts bar graphs representing approximate GAA activity as determined using the fluorescent substrate 4-methylumbelliferyl-α-glucoside. The Y-axes of panel A and panel B are moles of substrate hydrolyzed per mole of protein per hour. The X-axes list each GAA fusion protein.

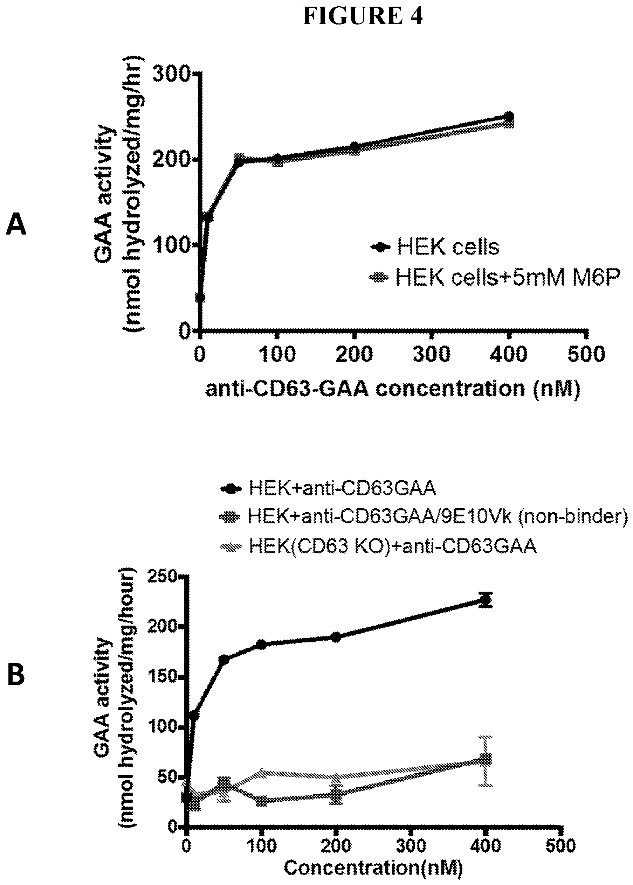

Figure 4 depicts line graphs representing approximate GAA activity of GAA constructs internalized by HEK cells. The Y-axes of panels A and B indicate nanomoles of substrate hydrolyzed per mg of cell lysate per hour. The X-axes indicate increasing concentration of the GAA construct. Panel A, squares (**■**) represent anti-CD63-GAA administered to HEK cells in the presence of 5 mM mannose-6-phosphate (M6P), a competitor of MPR-mediated lysosomal targeting, and circles (D) represent anti-CD63-GAA administered to HEK cells alone. Panel B, circles (D) represent anti-CD63-GAA administered to HEK cells, squares (**■**) represent a mutant anti-CD63-GAA, which does not bind to CD63, administered to HEK cells, and triangles (▲) represent anti-CD63-GAA administered to A CD63 HEK cells, which do not express CD63.

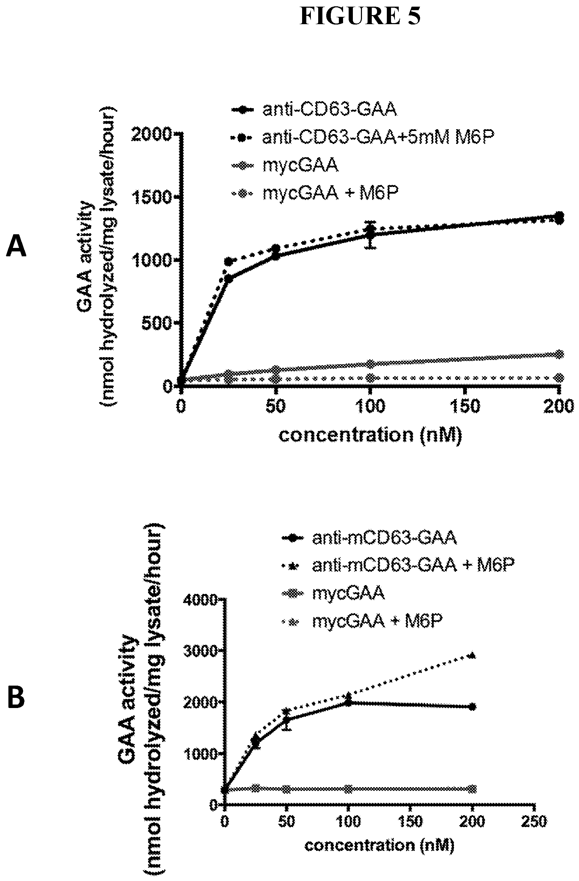

Figure 5 depicts line graphs representing approximate GAA activity of GAA constructs that were internalized by human (panel A) or murine (panel B) myoblasts. The Y-axes of panels A and B indicate nanomoles of substrate hydrolyzed per mg of cell lysate per hour. The X-axes indicate increasing concentration of the GAA construct, either anti-CD63-GAA or mycGAA, in the presence or absence of M6P.

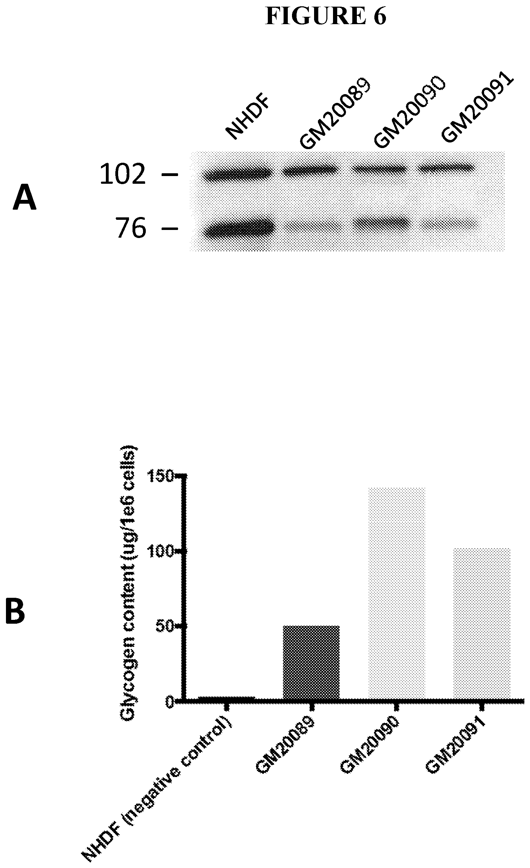

Figure 6 depicts GAA levels (panel A) and glycogen content (panel B) of three Pompe cell lines (GM20089, GM20090, and GM20091) compared to the GAA wild type neonatal human dermal fibroblasts (NHDF). Panel A is an anti-hGAA western blot depicting residual GAA protein in the Pompe cell lines and wildtype levels of GAA protein in NHDFs. Panel B is a bar graph depicting glycogen content as micrograms per million cells after glucose starvation to reduce cytoplasmic glycogen.

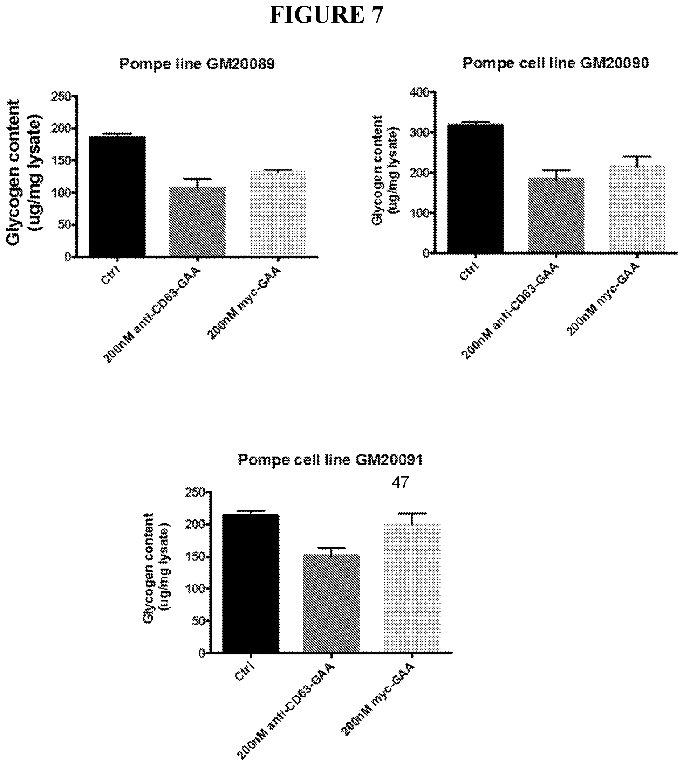

Figure 7 depicts in bar graph form the rescue of the glycogen accumulation defects for Pompe cell lines GM20089 (panel A), GM20090 (panel B), and GM20091 (panel C) by 200 nM anti-CD63-GAA or 200 nM myc-GAA. The Y-axis represents glycogen content in micrograms per milligram cell lysate.

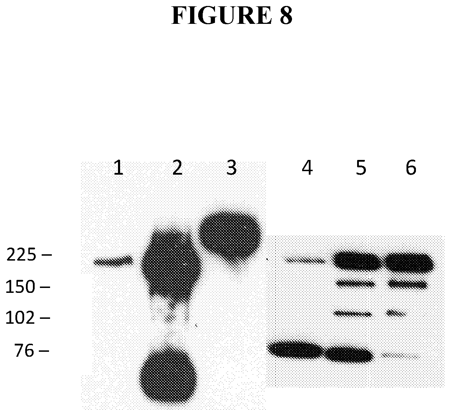

Figure 8 depicts an anti-hFc non-reduced western blot of CHO cell extract supernatants containing anti-CD63-GLA (lane 1), GLA-Fc knob (lane 2), GLA-anti-CD63 (lane 3), anti-myc knob (lane 4), anti-CD63 hole (lane 5), and mixture of supernatants containing anti-myc knob and anti-CD63 hole (lane 6).

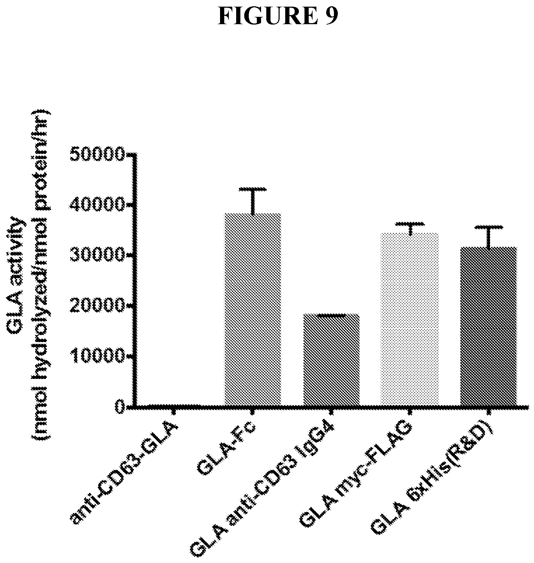

Figure 9 depicts in bar graph form the GLA enzymatic activity (Y-axis in nanomole substrate hydrolyzed per nanomole of fusion protein per hour) of GLA-containing fusion protein, including from left to right on the X-axis anti-CD63-GLA (the heavy chain C-terminus construct), GLA-Fc, GLA-anti-CD63 (the heavy chain N-terminus construct), GLA-myc-FLAG, and GLA 6-his.

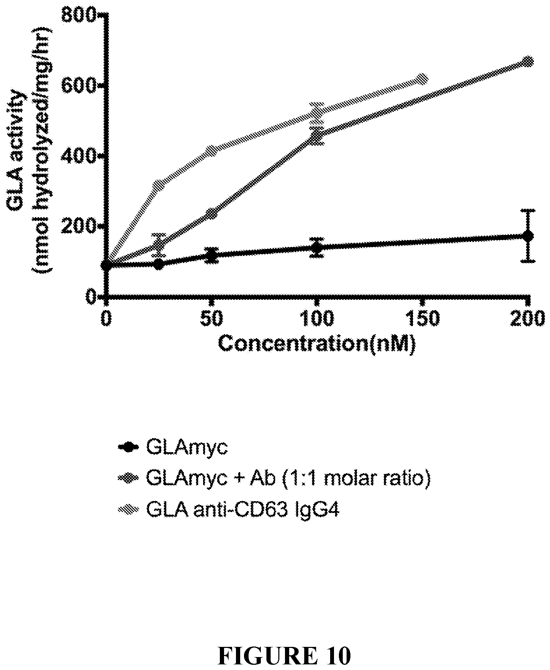

Figure 10 depicts a line graph representing approximate GLA activity found in extracts of HEK cells containing GLA constructs that were internalized by the HEK cells. The Y-axis indicates GLA activity in nanomoles of substrate hydrolyzed per milligram of cell lysate per hour. The X-axes indicate increasing concentration of the GAA construct. Top line represents GLA-anti-CD63, middle line represents GLA-myc plus anti-myc/anti-CD63 bispecific antibody, and bottom line represents GLA-myc.

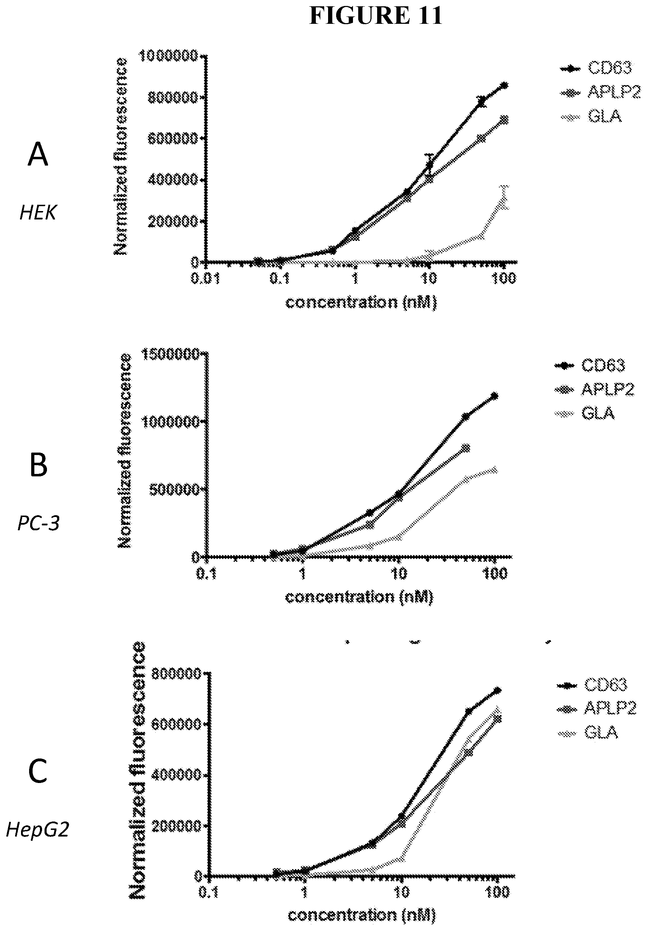

Figure 11 depicts line graphs representing the uptake of pHrodo-tagged proteins into the low pH fraction (*i.e.,* lysosomal fraction) of HEK cells (panel A), PC-3 cells (panel B), and HepG2 cells (panel 3). Circles (D) represent pHrodo-tagged anti-CD63 antibody, squares (**■**) represent pHrodo-tagged anti-APLP2 antibody, and triangles (**▲**) represent pHrodo-tagged GLA.

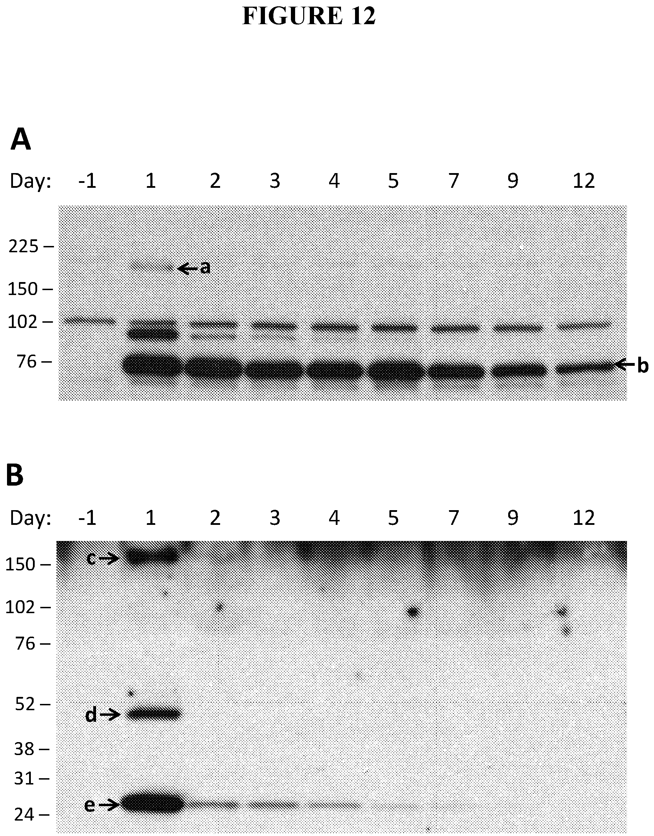

Figure 12 depicts reduced western blots of cell lysates containing internalized anti-CD63-GAA. Each lane represents cell extracts made at specific days post protein internalization. Panel A is a western blot probed with an anti-GAA antibody. The 150 kDa anti- CD63-GAA is visualized at marker ← a. The lysosomal 76 kDa active form of GAA is visualized at marker ← b . Panel B is a western blot probed with an anti-hIgG antibody. The 150 kDa anti-CD63-GAA is visualized at marker c →. The antibody heavy chain (50 kDa) is visualized at marker d →. The antibody light chain (23 kDa) is visualized at marker e →.

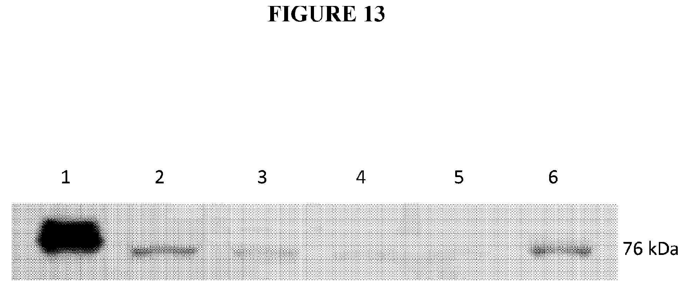

Figure 13 depicts an anti-hGAA antibody western. The 76 kDa band represents mature GAA. Lane 1 contains liver extracts from a humanized CD63 mouse given anti-CD63- GAA, lane 2 kidney, lane 3 heart, lane 4 gastrocnemius, lane 5 quadriceps, and lane 6 diaphragm.

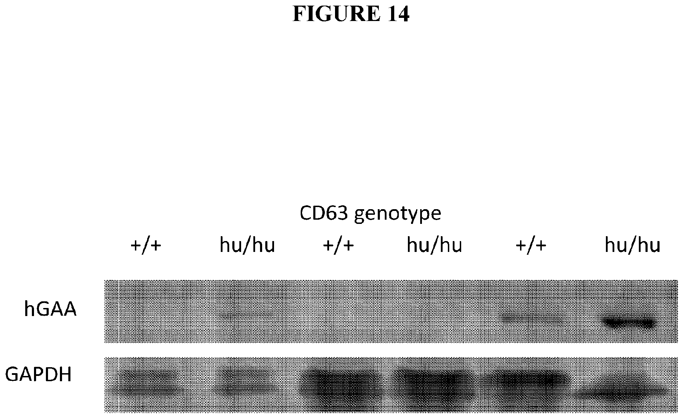

Figure 14 depicts an anti-hGAA antibody western blot of tissue extracts from a wild-type (+/+) mouse and humanized CD63 (hu/hu) mouse 24 hours after being given anti-hCD63-GAA at 50 mg/kg. The lysosomal 76 kDa active form of GAA is visualized. Lane 1 and 2 are heart extracts from wild-type and humanized mice, respectively. Lane 3 and 4 are gastrocnemius extracts from wild-type and humanized mice. Lane 5 and 6 are diaphragm extracts from wild-type and humanized mice.

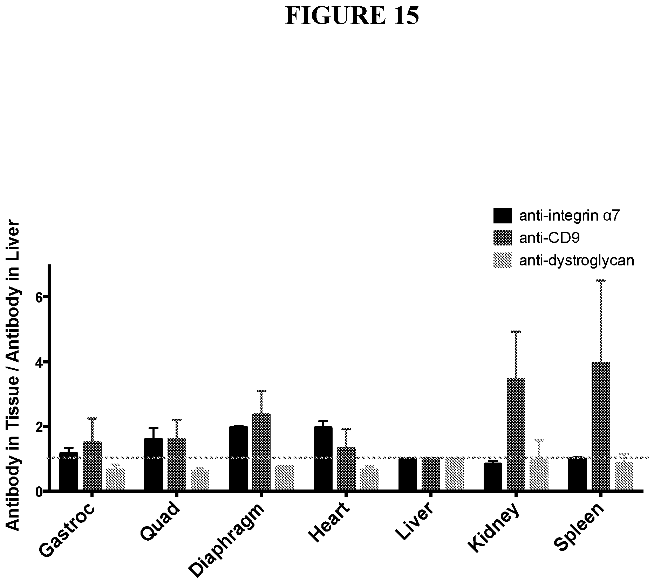

Figure 15 is a histogram depicting the relative amount of anti-integrin alpha 7 antibody, anti-CD9 antibody, and anti-dystroglycan antibody found within gastrocnemious muscle, quadriceps muscle, diaphragm, heart, liver, kidney, and spleen, normalized to levels found in liver.

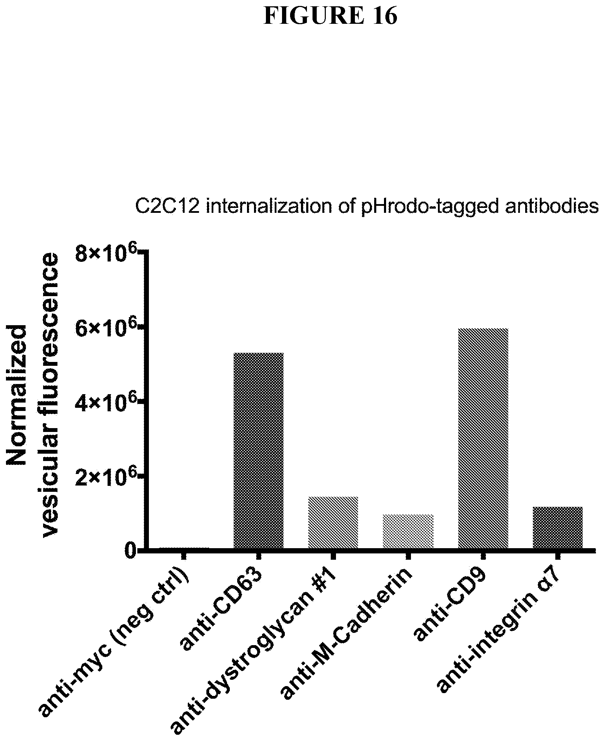

Figure 16 is a histogram representing the lysosomal targeting of pHrodo-tagged antibodies. The Y-axis represents normalized vesicular fluorescence. The X-axis represents each antibody, from left to right: anti-myc, anti-CD63, anti-dystroglycan, anti-M-cadherin, anti-CD9, and anti-integrin alpha 7.

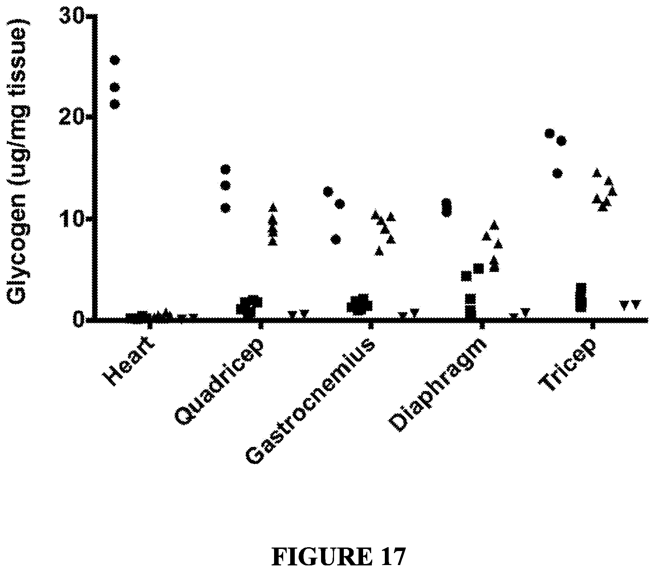

Figure 17 is a dot plot depicting glycogen levels expressed in micrograms of glycogen per milligram of tissue. Tissue is depicted on the X-axis from left to right as heart, quadriceps, gastrocnemius, diaphragm, and tricep. Circles (•) represent glycogen levels in untreated GAA knock-out (KO) mice, squares (**■**) represent glycogen levels in GAA KO mice treated with anti-mCD63-GAA, up triangles (A) represent glycogen levels in GAA KO mice treated with hGAA, and down triangle (▼) represent glycogen levels in untreated wildtype mice. Treatments were administered by hydrodynamic delivery of DNA constructs.

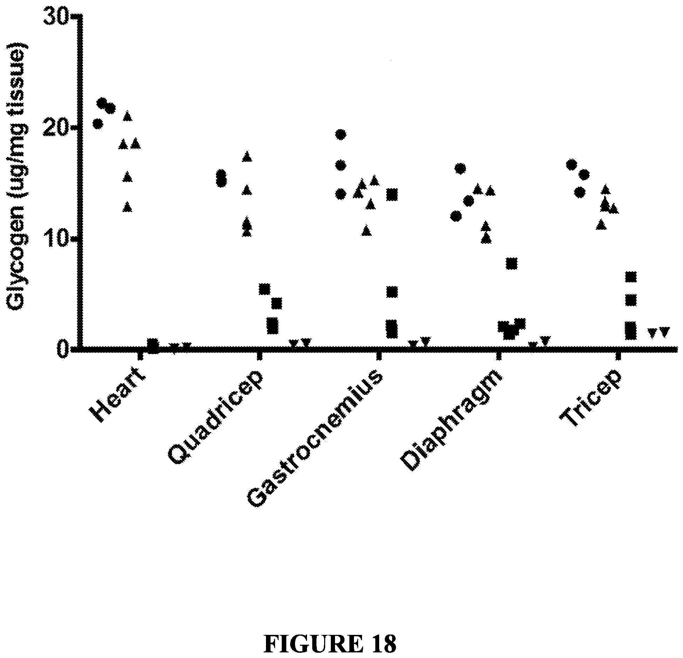

Figure 18 is a dot plot depicting glycogen levels expressed in micrograms of glycogen per milligram of tissue. Tissue is depicted on the X-axis from left to right as heart, quadriceps, gastrocnemius, diaphragm, and tricep. Circles (•) represent glycogen levels in untreated GAA knock-out (KO) mice, squares (**■**) represent glycogen levels in GAA KO mice treated with anti-mCD63-GAA, up triangles (A) represent glycogen levels in GAA KO mice treated with anti-hCD63-GAA, and down triangle (▼) represent glycogen levels in untreated wildtype mice. Treatments were administered by hydrodynamic delivery of DNA constructs.

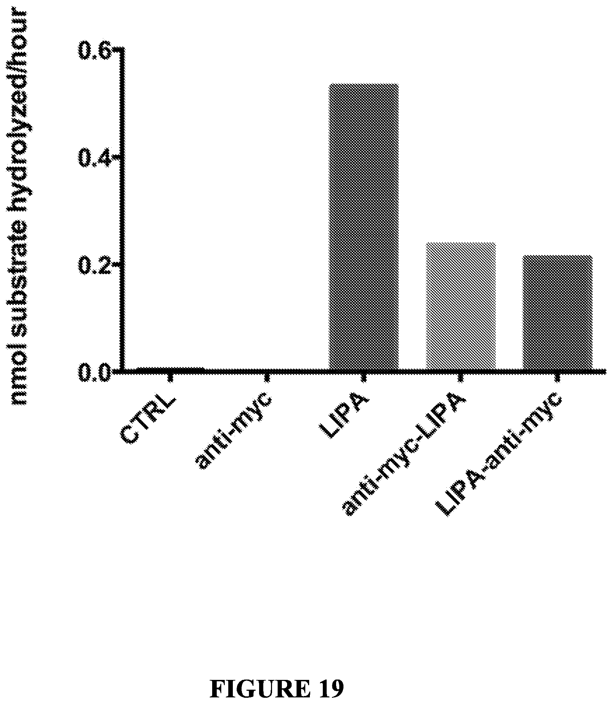

Figure 19 is a histogram depicting lipase activity expressed as nanomoles of substrate (4-methylumbelliferyl oleate) hydrolyzed per hour (Y-axis) by anti-myc antibody, native lysosomal acid lipase (LIPA), anti-myc-LIPA fusion protein (heavy chain C-terminal fusion), and LIPA-anti-myc (heavy chain N-terminal fusion).

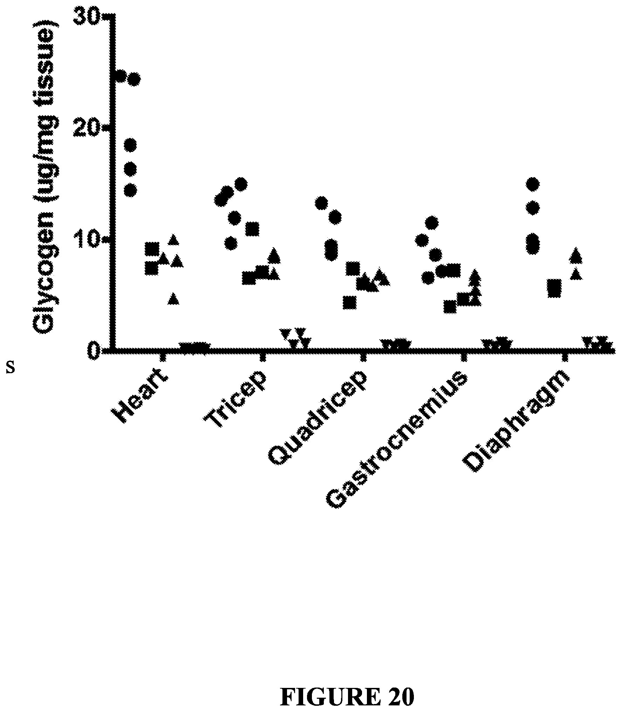

Figure 20 is a dot plot depicting glycogen levels expressed in micrograms of glycogen per milligram of tissue. Tissue is depicted on the X-axis from left to right as heart, tricep, quadricep, gastrocnemius and diaphragm. Circles (•) represent glycogen levels in untreated GAA knock-out (KO) mice, squares (**■**) represent glycogen levels in GAA KO mice treated with anti-mCD63-GAA, up triangles (A) represent glycogen levels in GAA KO mice treated with anti-hCD63-GAA, and down triangle (▼) represent glycogen levels in untreated wildtype mice. Treatments were administered by hydrodynamic delivery (HDD) of DNA constructs.

### DESCRIPTION

This invention is not limited to particular embodiments, compositions, methods and experimental conditions described, as such embodiments, compositions, methods and conditions may vary. The terminology used herein is for the purpose of describing particular embodiments only, and is not intended to be limiting, since the scope of the present invention will be limited only by the appended claims.

Although any methods and materials similar or equivalent to those described herein can be used in the practice or testing of the present invention, some preferred methods and materials are now described. Unless defined otherwise, all technical and scientific terms used herein have the same meaning as commonly understood by one of ordinary skill in the art to which this invention belongs.

"Lysosomal storage diseases" include any disorder resulting from a defect in lysosome function. Currently, approximately 50 disorders have been identified, the most well-known of which include Tay-Sachs, Gaucher, and Niemann-Pick disease. The pathogeneses of the diseases are ascribed to the buildup of incomplete degradation products in the lysosome, usually due to loss of protein function. Lysosomal storage diseases are caused by loss-of-function or attenuating variants in the proteins whose normal function is to degrade or coordinate degradation of lysosomal contents. The proteins affiliated with lysosomal storage diseases include enzymes, receptors and other transmembrane proteins (*e.g.,* NPC1), post-translational modifying proteins (*e.g.,* sulfatase), membrane transport proteins, and non-enzymatic cofactors and other soluble proteins (*e.g.*, GM2 ganglioside activator). Thus, lysosomal storage diseases encompass more than those disorders caused by defective enzymes *per se,* and include any disorder caused by any molecular defect. Thus, as used herein, the term "enzyme" is meant to encompass those other proteins associated with lysosomal storage diseases.

The nature of the molecular lesion affects the severity of the disease in many cases, *i.e.* complete loss-of-function tends to be associated with pre-natal or neo-natal onset, and involves severe symptoms; partial loss-of-function is associated with milder (relatively) and later-onset disease. Generally, only a small percentage of activity needs to be restored to have to correct metabolic defects in deficient cells. Table 1 lists some of the more common lysosomal storage diseases and their associated loss-of-function proteins. Lysosomal storage diseases are generally described in Desnick and Schuchman, 2012.

Lysosomal storage diseases can be categorized according to the type of product that accumulates within the defective lysosome. Sphingolipidoses are a class of diseases that affect the metabolism of sphingolipids, which are lipids containing fatty acids linked to aliphatic amino alcohols (reviewed in S. Hakomori, "Glycosphingolipids in Cellular Interaction, Differentiation, and Oncogenesis," 50 Annual Review of Biochemistry 733-764, July 1981). The accumulated products of sphingolipidoses include gangliosides (*e.g.,* Tay-Sachs disease), glycolipids (*e.g.,* Fabry's disease), and glucocerebrosides (*e.g.*, Gaucher's disease).

Mucopolysaccharidoses are a group of diseases that affect the metabolism of glycosaminoglycans (GAGS or mucopolysaccharides), which are long unbranched chains of repeating disaccharides that help build bone, cartilage, tendons, corneas, skin and connective tissue (reviewed in J. Muenzer, "Early initiation of enzyme replacement therapy for the mucopolysaccharidoses," 111(2) Mol. Genet. Metab. 63-72 (Feb. 2014); Sasisekharan et al., "Glycomics approach to structure-function relationships of glycosaminoglycans," 8(1) Ann. Rev. Biomed. Eng. 181-231 (Dec. 2014)). The accumulated products of mucopolysaccharidoses include heparan sulfate, dermatan sulfate, keratin sulfate, various forms of chondroitin sulfate, and hyaluronic acid. For example, Morquio syndrome A is due to a defect in the lysosomal enzyme galactose-6-sulfate sulfatase, which results in the lysosomal accumulation of keratin sulfate and chondroitin 6-sulfate.

Glycogen storage diseases (a.k.a., glycogenosis) result from a cell's inability to metabolize (make or break-down) glycogen. Glycogen metabolism is moderated by various enzymes or other proteins including glucose-6-phosphatase, acid alpha-glucosidase, glycogen de-branching enzyme, glycogen branching enzyme, muscle glycogen phosphorylase, liver glycogen phosphorylase, muscle phosphofructokinase, phosphorylase kinase, glucose transporter, aldolase A, beta-enolase, and glycogen synthase. An exemplar lysosomal storage/glycogen storage disease is Pompe's disease, in which defective acid alpha-glucosidase causes glycogen to accumulate in lysosomes. Symptoms include hepatomegaly, muscle weakness, heart failure, and in the case of the infantile variant, death by age 2 (see DiMauro and Spiegel, "Progress and problems in muscle glycogenosis," 30(2) Acta Myol. 96-102 (Oct. 2011)).

"Biotherapeutic complex" includes (i) a single protein that contains more than one functional domain, (ii) a protein that contains more than one polypeptide chain, and (iii) a mixture of more than one protein or more than one polypeptide. The term polypeptide is generally taken to mean a single chain of amino acids linked together via peptide bonds. The term protein encompasses the term polypeptide, but also includes more complex structures. That is, a single polypeptide is a protein, and a protein can contain one or more polypeptides associated in a higher order structure. For example, hemoglobin is a protein containing four polypeptides: two alpha globin polypeptides and two beta globin polypeptides. Myoglobin is also a protein, but it contains only a single myoglobin polypeptide.

The biotherapeutic complex comprises one or more polypeptide(s) and at least two functions. One of those functions is the replacement of a defective protein activity associated with a lysosomal storage disease. The other of those functions is the binding to an internalization effector. Thus, a single polypeptide that provides a lysosomal protein activity (*e.g.*, an enzymatic activity or transporter activity; a.k.a. lysosomal disease-related protein (LSD-RP) activity) and the ability to bind to an internalization effector (a.k.a. internalization effector-binding protein (IE-BP activity) is a biotherapeutic complex. Also, a mixture of proteins, wherein one protein has the lysosomal protein function, and another protein has the internalization effector binding activity, is a biotherapeutic complex. Figure 1 depicts various exemplars of biotherapeutic complexes. In one example (Figure 1, panel A), the biotherapeutic complex contains a lysosomal replacement protein (the LSD-RP represented by the hexagon) and a bispecific antibody (the 1E-BP) that binds the lysosomal replacement protein (hashed lines) and an internalization effector (solid lines). Here, one arm of the bispecific antibody binds non-covalently to the LSD-RP, and the other arm binds non-covalently to the internalization effector, thereby enabling the internalization of the replacement protein (LSD-RP) into the lysosome. In another example (panel B), the biotherapeutic complex comprises a single protein containing two polypeptides, one polypeptide having LSD-RP function and the other having IE-BP function. Here, the LSD-RP is fused to an immunoglobulin Fc domain or heavy chain constant region, which associates with the Fc domain of the LSD-RP half-antibody to form the bifunctional biotherapeutic complex. The example depicted in panel B is similar to that in panel A, except that the LSD-RP is covalently attached to one of the half-antibodies, rather than through antigen-antibody interaction at the immunoglobulin variable domain of the half-antibody.

In other examples, the biotherapeutic complex consists of the LSD-RP covalently linked (directly or indirectly through a linker) to the IE-BP. In one aspect, the LSD-RP is attached to the C-terminus of an immunoglobulin molecule (*e.g.,* the heavy chain or alternatively the light chain). In another aspect, the LSD-RP is attached to the N-terminus of the immunoglobulin molecule (*e.g.,* the heavy chain or alternatively the light chain). In these exemplars, the immunoglobulin molecule is the IE-BP.

"Lysosomal storage disease-related protein" or "LSD-RP" denotes any protein associated with the etiology or physiological effect of a lysosomal storage disease. An LSD-RP includes the actual enzyme, transport protein, receptor, or other protein that is defective and which is attributed as the molecular lesion that caused the disease. An LSD-RP also includes any protein that can provide a similar or sufficient biochemical or physiological activity that replaces or circumvents the molecular lesion of the disease. For example, an "isozyme" may be used as an LSD-RP. Examples of lysosomal storage disease-related proteins include those listed in Table 1 as "Involved Enzyme/Protein" and any known or later discovered protein or other molecule that circumvents the molecular defect of the lysosomal storage disease.

In the case of Pompe disease, in which the molecular defect is a defect in α-glucosidase activity, LSD-RPs include human alpha-glucosidase, and "isozymes" such as other alpha-glucosidases, engineered recombinant alpha-glucosidase, other glucosidases, recombinant glucosidases, any protein engineered to hydrolyze a terminal non-reducing 1-4 linked alpha-glucose residue to release a single alpha- glucose molecule, any EC 3.2.1.20 enzyme, natural or recombinant low pH carbohydrate hydrolases for glycogen or starches, and glucosyl hydrolases such as sucrase isomaltase, maltase glucoamylase, glucosidase II, and neutral alpha-glucosidase.

In the compositions of the present invention, the composition comprises an enzyme associated with the LSD, wherein the enzyme is α-glucosidase (GAA).

Similarly, in the biotherapeutic complexes provided for use in the invention the biotherapeutic complexes comprise a replacement enzyme which is α-glucosidase (GAA).

An "internalizing effector" includes a protein that is capable of being internalized into a cell or that otherwise participates in or contributes to retrograde membrane trafficking. In some instances, the internalizing effector is a protein that undergoes transcytosis; that is, the protein is internalized on one side of a cell and transported to the other side of the cell (*e.g.*, apical-to-basal). In many aspects, the internalizing effector protein is a cell surface-expressed protein or a soluble extracellular protein. However, the present disclosure also contemplates aspects in which the internalizing effector protein is expressed within an intracellular compartment such as the endosome, endoplasmic reticulum, Golgi, lysosome, *etc.* For example, proteins involved in retrograde membrane trafficking (*e.g.*, pathways from early/recycling endosomes to the trans-Golgi network) may serve as internalizing effector proteins in various aspects of the present disclosure. In any event, the binding of the IE-BP to an internalizing effector protein causes the entire biotherapeutic complex, and any molecules associated therewith (*e.g.*, LSD-RP), to also become internalized into the cell. As explained below, internalizing effector proteins include proteins that are directly internalized into a cell, as well as proteins that are indirectly internalized into a cell.

Internalizing effector proteins that are directly internalized into a cell include membrane-associated molecules with at least one extracellular domain (*e.g.*, transmembrane proteins, GPI-anchored proteins, etc.), which undergo cellular internalization, and are preferably processed via an intracellular degradative and/or recycling pathway. Specific non-limiting examples of internalizing effector proteins that are directly internalized into a cell include, *e.g.,* CD63, MHC-I (*e.g.,* HLA-B27), Kremen-1, Kremen-2, LRP5, LRP6, LRP8, transferrin receptor, LDL-receptor, LDL-related protein 1 receptor, ASGR1, ASGR2, amyloid precursor protein-like protein-2 (APLP2), apelin receptor (APLNR), MAL (Myelin And Lymphocyte protein, a.k.a. VIP17), IGF2R, vacuolar-type H+ ATPase, diphtheria toxin receptor, folate receptor, glutamate receptors, glutathione receptor, leptin receptors, scavenger receptors (*e.g.*, SCARA1-5, SCARB1-3, CD36), and the like.

In certain aspects, the internalizing effector is prolactin receptor (PRLR). It was discovered that PRLR is, not only a target for certain therapeutic applications, but is also an effective internalizing effector protein on the basis of its high rate of internalization and turn-over. The potential for PRLR as an internalizing effector protein, for example, is illustrated in WO2015/026907, where it is demonstrated, *inter alia,* that anti-PRLR antibodies are effectively internalized by PRLR-expressing cells in vitro.

In certain aspects, the internalization effector is a kidney specific internalizer, such as CDH16 (Cadheri-16), CLDN16 (Claudn-16), KL (Klotho), PTH1R (parathyroid hormone receptor), SLC22A13 (Solute carrier family 22 member 13), SLC5A2 (Sodium/glucose cotransporter 2), and UMOD (Uromodulin). In other certain aspects, the internalization effector is a muscle specific internalizer, such as BMPR1A (Bone morphogenetic protein receptor 1A), m-cadherin, CD9, MuSK (muscle-specific kinase), LGR4/GPR48 (G protein-coupled receptor 48), cholinergic receptor (nicotinic) alpha 1, CDH15 (Cadheri-15), ITGA7 (Integrin alpha-7), CACNG1 (L-type calcium channel subunit gamma-1), CACNAlS (L-type calcium channel subunit alpha-15), CACNG6 (L-type calcium channel subunit gamma-6), SCN1B (Sodium channel subunit beta-1), CHRNA1 (ACh receptor subunit alpha), CHRND (ACh receptor subunit delta), LRRC14B (Leucine-rich repeat-containing protein 14B), and POPDC3 (Popeye domain-containing protein 3),In some specific aspects, the internalization effector is ITGA7, CD9, CD63, ALPL2, or PPRLR. In the present invention, the internalization effector is transferrin receptor.

In those aspects in which the internalization effector (IE) is directly internalized into a cell, the IE-BP can be, *e.g.,* an antibody or antigen-binding fragment of an antibody that specifically binds the IE, or a ligand or portion of a ligand that specifically interacts with the IE. For example, if the IE is Kremen-1 or Kremen-2, the IE-BP can comprise or consist of a Kremen ligand (*e.g.,* DKK1) or Kremen-binding portion thereof. As another example, if the IE is a receptor molecule such as ASGR1, the IE-BP can comprise or consist of a ligand specific for the receptor (*e.g.,* asialoorosomucoid [ASOR] or Beta-Ga1NAc) or a receptor-binding portion thereof.

Internalizing effector proteins that are indirectly internalized into a cell include proteins and polypeptides that do not internalize on their own, but become internalized into a cell after binding to or otherwise associating with a second protein or polypeptide that is directly internalized into the cell. Proteins that are indirectly internalized into a cell include, *e.g.,* soluble ligands that are capable of binding to an internalizing cell surface-expressed receptor molecule. A non-limiting example of a soluble ligand that is (indirectly) internalized into a cell via its interaction with an internalizing cell surface-expressed receptor molecule is transferrin. In aspects wherein the IE is transferrin (or another indirectly internalized protein), the binding of the IE-BP to the IE, and the interaction of IE with transferrin receptor (or another internalizing cell-surface expressed receptor molecule), causes the entire IE-BP, and any molecules associated therewith (*e.g.,* the LSD-RP), to become internalized into the cell concurrent with the internalization of the IE and its binding partner.

In those aspects in which the IE is indirectly internalized into a cell, the IE- BP can be, *e.g.,* an antibody or antigen-binding fragment of an antibody that specifically binds IE, or a receptor or portion of a receptor that specifically interacts with the soluble effector protein. For example, if the IE is a cytokine, the IE-BP can comprise or consist of the corresponding cytokine receptor or ligand-binding portion thereof.

An exemplar IE is CD63, which is a member of the tetraspanin superfamily of cell surface proteins that span the cell membrane four times. CD63 is expressed in virtually all tissues and is thought to be involved in forming and stabilizing signaling complexes. CD63 localizes to the cell membrane, lysosomal membrane, and late endosomal membrane. CD63 is known to associate with integrins and may be involved in epithelial-mesenchymal transitioning. See H. Maecker et al., "The tetraspanin superfamily: molecular facilitators," 11(6) FASEB J. 428-42, May 1997; and M. Metzelaar et al., "CD63 antigen. A novel lysosomal membrane glycoprotein, cloned by a screening procedure for intracellular antigens in eukaryotic cells," 266 J. Biol. Chem. 3239-3245, 1991.

Another exemplar IE is amyloid beta (A4) precursor-like protein 2 ("APLP2"), a ubiquitously expressed member of the APP (amyloid precursor protein) family. APLP2 is a membrane-bound protein known to interact with major histocompatibility complex (MHC) class I molecules (*e.g.,* Kd ). It binds Kd at the cell surface and is internalized in a clathri*N*-dependent manner with Kd in tow. See Tuli et al., "Mechanism for amyloid precursor-like protein 2 enhancement of major histocompatibility complex class I molecule degradation," 284 The Journal of Biological Chemistry 34296 -34307 (2009).

Another IE exemplar is the prolactin receptor (PRLR). The prolactin receptor is a member of the type I cytokine receptor family and upon ligand binding and subsequent dimerization activates "the tyrosine kinases Jak2, Fyn and Tec, the phosphatase SHP-2, the guanine nucleotide exchange factor Vav, and the signaling suppressor SOCS," (see Clevenger and Kline, "Prolactin receptor signal transduction," 10(10) Lupus 706-18 (2001), abstract). The prolactin receptor undergoes endocytotic recycling and can be found in lysosomal fractions. See Genty et al., "Endocytosis and degradation of prolactin and its receptor in Chinese hamster ovary cells stably transfected with prolactin receptor cDNA," 99(2) Mol. Cell Endocrinol. 221-8 (1994); and Ferland et al., "The effect of chloroquine on lysosomal prolactin receptors in rat liver," 115(5) Endocrinology 1842-9 (1984).

As used herein, "immunological reaction" generally means a patient's immunological response to an outside or "non-self protein. This immunological response includes an allergic reaction and the development of antibodies that interfere with the effectiveness of the replacement enzyme. Some patients may not produce any of the non-functioning protein, thus rendering the replacement enzyme a "foreign" protein. For example, repeated injection of recombinant GLA (rGLA) to those Fabry patients who lack GLA frequently results in an allergic reaction. In other patients, the production of antibodies against rGLA has been shown to decrease the effectiveness of the replacement enzyme in treating the disease. See for example Tajima et al. ("Use of a Modified α-N-Acetylgalactosaminidase (NAGA) in the Development of Enzyme Replacement Therapy for Fabry Disease," 85(5) Am. J. Hum. Genet. 569-580 (2009)), which discusses the use of modified NAGA as the "isozyme" to replace GLA. The modified NAGA has no immunological cross-reactivity with GLA, and "did not react to serum from a patient with Fabry disease recurrently treated with a recombinant GLA." *Id,* abstract

The term "protein" means any amino acid polymer having more than about 20 amino acids covalently linked via amide bonds. Proteins contain one or more amino acid polymer chains, generally known in the art as "polypeptides". Thus, a polypeptide may be a protein, and a protein may contain multiple polypeptides to form a single functioning biomolecule. Disulfide bridges (*i.e.,* between cysteine residues to form cystine) may be present in some proteins. These covalent links may be within a single polypeptide chain, or between two individual polypeptide chains. For example, disulfide bridges are essential to proper structure and function of insulin, immunoglobulins, protamine, and the like. For a recent review of disulfide bond formation, see Oka and Bulleid, "Forming disulfides in the endoplasmic reticulum," 1833(11) Biochim Biophys Acta 2425-9 (2013).

In addition to disulfide bond formation, proteins may be subject to other post- translational modifications. Those modifications include lipidation (*e.g.*, myristoylation, palmitoylation, farnesoylation, geranylgeranylation, and glycosylphosphatidylinositol (GPI) anchor formation), alkylation (*e.g.,* methylation), acylation, amidation, glycosylation (*e.g.,* addition of glycosyl groups at arginine, asparagine, cysteine, hydroxylysine, serine, threonine, tyrosine, and/or tryptophan), and phosphorylation (*i.e.,* the addition of a phosphate group to serine, threonine, tyrosine, and/or histidine). For a recent review on the post-translational modification of proteins produced in eukaryotes, see Mowen and David, "Unconventional post-translational modifications in immunological signaling," 15(6) Nat Immunol 512-20 (2014); and Blixt and Westerlind, "Arraying the post-translational glycoproteome (PTG)," 18 Curr Opin Chem Biol. 62-9 (2014).

Immunoglobulins are proteins having multiple polypeptide chains and extensive post-translational modifications. The canonical immunoglobulin protein (*e.g.,* IgG) comprises four polypeptide chains - two light chains and two heavy chains. Each light chain is linked to one heavy chain via a cystine disulfide bond, and the two heavy chains are bound to each other via two cystine disulfide bonds. Immunoglobulins produced in mammalian systems are also glycosylated at various residues (*e.g.*, at asparagine residues) with various polysaccharides, and can differ from species to species, which may affect antigenicity for therapeutic antibodies (see Butler and Spearman, "The choice of mammalian cell host and possibilities for glycosylation engineering", 30 Curr Opin Biotech 107-112 (2014)).

As used herein, "protein" includes biotherapeutic proteins, recombinant proteins used in research or therapy, trap proteins and other Fc-fusion proteins, chimeric proteins, antibodies, monoclonal antibodies, human antibodies, bispecific antibodies, antibody fragments, nanobodies, recombinant antibody chimeras, cytokines, chemokines, peptide hormones, and the like. Proteins may be produced using recombinant cell-based production systems, such as the insect bacculovirus system, yeast systems (*e.g.,* Pichia sp.), mammalian systems (*e.g.,* CHO cells and CHO derivatives like CHO-K1 cells). For a recent review discussing biotherapeutic proteins and their production, see Ghaderi et al., "Production platforms for biotherapeutic glycoproteins. Occurrence, impact, and challenges of non-human sialylation," 28 Biotechnol Genet Eng Rev. 147-75 (2012).

The term "antibody", as used herein, includes immunoglobulin molecules comprising four polypeptide chains, two heavy (H) chains and two light (L) chains inter-connected by disulfide bonds. Each heavy chain comprises a heavy chain variable region (abbreviated herein as HCVR or VH) and a heavy chain constant region. The heavy chain constant region comprises three domains, CH1, CH2 and CH3. Each light chain comprises a light chain variable region (abbreviated herein as LCVR or VL) and a light chain constant region. The light chain constant region comprises one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4 (heavy chain CDRs may be abbreviated as HCDR1, HCDR2 and HCDR3; light chain CDRs may be abbreviated as LCDR1, LCDR2 and LCDR3. The term "high affinity" antibody refers to those antibodies having a binding affinity to their target of at least 10⁻⁹ M, at least 10⁻¹⁰ M; at least 10⁻¹¹ M; or at least 10⁻¹² M, as measured by surface plasmon resonance, *e.g.,* BIACORE^{™} or solution-affinity ELISA.

The phrase "bispecific antibody" includes an antibody capable of selectively binding two or more epitopes. Bispecific antibodies generally comprise two different heavy chains, with each heavy chain specifically binding a different epitope-either on two different molecules (*e.g.*, antigens) or on the same molecule (*e.g.,* on the same antigen). If a bispecific antibody is capable of selectively binding two different epitopes (a first epitope and a second epitope), the affinity of the first heavy chain for the first epitope will generally be at least one to two or three or four orders of magnitude lower than the affinity of the first heavy chain for the second epitope, and vice versa. The epitopes recognized by the bispecific antibody can be on the same or a different target (*e.g.,* on the same or a different protein). Bispecific antibodies can be made, for example, by combining heavy chains that recognize different epitopes of the same antigen. For example, nucleic acid sequences encoding heavy chain variable sequences that recognize different epitopes of the same antigen can be fused to nucleic acid sequences encoding different heavy chain constant regions, and such sequences can be expressed in a cell that expresses an immunoglobulin light chain. A typical bispecific antibody has two heavy chains each having three heavy chain CDRs, followed by (N-terminal to C-terminal) a CH1 domain, a hinge, a CH2 domain, and a CH3 domain, and an immunoglobulin light chain that either does not confer antigen-binding specificity but that can associate with each heavy chain, or that can associate with each heavy chain and that can bind one or more of the epitopes bound by the heavy chain antigen-binding regions, or that can associate with each heavy chain and enable binding or one or both of the heavy chains to one or both epitopes.

The phrase "heavy chain," or "immunoglobulin heavy chain" includes an immunoglobulin heavy chain constant region sequence from any organism, and unless otherwise specified includes a heavy chain variable domain. Heavy chain variable domains include three heavy chain CDRs and four FR regions, unless otherwise specified. Fragments of heavy chains include CDRs, CDRs and FRs, and combinations thereof. A typical heavy chain has, following the variable domain (from N-terminal to C-terminal), a CH1 domain, a hinge, a CH2 domain, and a CH3 domain. A functional fragment of a heavy chain includes a fragment that is capable of specifically recognizing an antigen (*e.g.*, recognizing the antigen with a KD in the micromolar, nanomolar, or picomolar range), that is capable of expressing and secreting from a cell, and that comprises at least one CDR.

The phrase "light chain" includes an immunoglobulin light chain constant region sequence from any organism, and unless otherwise specified includes human kappa and lambda light chains. Light chain variable (VL) domains typically include three light chain CDRs and four framework (FR) regions, unless otherwise specified. Generally, a full-length light chain includes, from amino terminus to carboxyl terminus, a VL domain that includes FR1-CDR1- FR2-CDR2-FR3-CDR3-FR4, and a light chain constant domain. Light chains that can be used include *e.g.,* those, that do not selectively bind either the first or second antigen selectively bound by the antigen-binding protein. Suitable light chains include those that can be identified by screening for the most commonly employed light chains in existing antibody libraries (wet libraries or *in silico*), where the light chains do not substantially interfere with the affinity and/or selectivity of the antigen-binding domains of the antigen-binding proteins. Suitable light chains include those that can bind one or both epitopes that are bound by the antigen-binding regions of the antigen-binding protein.

The phrase "variable domain" includes an amino acid sequence of an immunoglobulin light or heavy chain (modified as desired) that comprises the following amino acid regions, in sequence from N-terminal to C-terminal (unless otherwise indicated): FR1, CDR1, FR2, CDR2, FR3, CDR3, FR4. A "variable domain" includes an amino acid sequence capable of folding into a canonical domain (VH or VL) having a dual beta sheet structure wherein the beta sheets are connected by a disulfide bond between a residue of a first beta sheet and a second beta sheet.

The phrase "complementarity determining region," or the term "CDR," includes an amino acid sequence encoded by a nucleic acid sequence of an organism's immunoglobulin genes that normally (*i.e.*, in a wild-type animal) appears between two framework regions in a variable region of a light or a heavy chain of an immunoglobulin molecule (*e.g.,* an antibody or a T cell receptor). A CDR can be encoded by, for example, a germline sequence or a rearranged or unrearranged sequence, and, for example, by a naive or a mature B cell or a T cell. In some circumstances (*e.g.*, for a CDR3), CDRs can be encoded by two or more sequences (*e.g.*, germline sequences) that are not contiguous (*e.g.*, in an unrearranged nucleic acid sequence) but are contiguous in a B cell nucleic acid sequence, *e.g.,* as the result of splicing or connecting the sequences (*e.g.,* V-D-J recombination to form a heavy chain CDR3).

The term "antibody fragment", refers to one or more fragments of an antibody that retain the ability to specifically bind to an antigen. Examples of binding fragments encompassed within the term "antibody fragment" include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al. (1989) Nature 241:544-546), which consists of a VH domain, (vi) an isolated CDR, and (vii) an scFv, which consists of the two domains of the Fv fragment, VL and VH, joined by a synthetic linker to form a single protein chain in which the VL and VH regions pair to form monovalent molecules. Other forms of single chain antibodies, such as diabodies are also encompassed under the term "antibody" (see *e.g.,* Holliger et al. (1993) PNAS USA 90:6444-6448; Poljak et al. (1994) Structure 2:1121-1123).

The phrase "Fc-containing protein" includes antibodies, bispecific antibodies, immunoadhesins, and other binding proteins that comprise at least a functional portion of an immunoglobulin CH2 and CH3 region. A "functional portion" refers to a CH2 and CH3 region that can bind a Fc receptor (*e.g.,* an FcyR; or an FcRn, *i.e.,* a neonatal Fc receptor), and/or that can participate in the activation of complement. If the CH2 and CH3 region contains deletions, substitutions, and/or insertions or other modifications that render it unable to bind any Fc receptor and also unable to activate complement, the CH2 and CH3 region is not functional.

Fc-containing proteins can comprise modifications in immunoglobulin domains, including where the modifications affect one or more effector function of the binding protein (*e.g.,* modifications that affect FcyR binding, FcRn binding and thus half-life, and/or CDC activity). Such modifications include, but are not limited to, the following modifications and combinations thereof, with reference to EU numbering of an immunoglobulin constant region: 238, 239, 248, 249, 250, 252, 254, 255, 256, 258, 265, 267, 268, 269, 270, 272, 276, 278, 280, 283, 285, 286, 289, 290, 292, 293, 294, 295, 296, 297, 298, 301, 303, 305, 307, 308, 309, 311, 312, 315, 318, 320, 322, 324, 326, 327, 328, 329, 330, 331, 332, 333, 334, 335, 337, 338, 339, 340, 342, 344, 356, 358, 359, 360, 361, 362, 373, 375, 376, 378, 380, 382, 383, 384, 386, 388, 389, 398, 414, 416, 419, 428, 430, 433, 434, 435, 437, 438, and 439.

For example, and not by way of limitation, the binding protein is an Fc-containing protein and exhibits enhanced serum half-life (as compared with the same Fc-containing protein without the recited modification(s)) and have a modification at position 250 (*e.g.,* E or Q); 250 and 428 (*e.g.,* L or F); 252 (*e.g.,* L/Y/F/W or T), 254 (*e.g.,* S or T), and 256 (*e.g.,* S/R/Q/E/D or T); or a modification at 428 and/or 433 (*e.g.,* L/R/SI/P/Q or K) and/or 434 (*e.g.,* H/F or Y); or a modification at 250 and/or 428; or a modification at 307 or 308 (*e.g.,* 308F, V308F), and 434. In another example, the modification can comprise a 428L (*e.g.,* M428L) and 434S (*e.g.,* N434S) modification; a 428L, 2591 (*e.g.,* V259I), and a 308F (*e.g.,* V308F) modification; a 433K (*e.g.,* H433K) and a 434 (*e.g.,* 434Y) modification; a 252, 254, and 256 (*e.g.,* 252Y, 254T, and 256E) modification; a 250Q and 428L modification (*e.g.*, T250Q and M428L); a 307 and/or 308 modification (*e.g.*, 308F or 308P).

The term "antigen-binding protein," as used herein, refers to a polypeptide or protein (one or more polypeptides complexed in a functional unit) that specifically recognizes an epitope on an antigen, such as a cell-specific antigen and/or a target antigen. An antigen-binding protein may be multi-specific. The term "multi-specific" with reference to an antigen-binding protein means that the protein recognizes different epitopes, either on the same antigen or on different antigens. A multi-specific antigen-binding protein can be a single multifunctional polypeptide, or it can be a multimeric complex of two or more polypeptides that are covalently or non-covalently associated with one another. The term "antigen-binding protein" includes antibodies or fragments thereof that may be linked to or co-expressed with another functional molecule, *e.g.*, another peptide or protein. For example, an antibody or fragment thereof can be functionally linked (*e.g.,* by chemical coupling, genetic fusion, non-covalent association or otherwise) to one or more other molecular entities, such as a protein or fragment thereof to produce a bispecific or a multi-specific antigen-binding molecule with a second binding specificity.

As used herein, the term "epitope" refers to the portion of the antigen which is recognized by the multi-specific antigen-binding polypeptide. A single antigen (such as an antigenic polypeptide) may have more than one epitope. Epitopes may be defined as structural or functional. Functional epitopes are generally a subset of structural epitopes and are defined as those residues that directly contribute to the affinity of the interaction between the antigen-binding polypeptide and the antigen. Epitopes may also be conformational, that is, composed of non-linear amino acids. In certain aspects, epitopes may include determinants that are chemically active surface groupings of molecules such as amino acids, sugar side chains, phosphoryl groups, or sulfonyl groups, and, in certain aspects, may have specific three-dimensional structural characteristics, and/or specific charge characteristics. Epitopes formed from contiguous amino acids are typically retained on exposure to denaturing solvents, whereas epitopes formed by tertiary folding are typically lost on treatment with denaturing solvents.

The term "domain" refers to any part of a protein or polypeptide having a particular function or structure. Preferably, domains of the present invention bind to cell-specific or target antigens. Cell-specific antigen- or target antigen-binding domains, and the like, as used herein, include any naturally occurring, enzymatically obtainable, synthetic, or genetically engineered polypeptide or glycoprotein that specifically binds an antigen.

The term "half-body" or "half-antibody", which are used interchangeably, refers to half of an antibody, which essentially contains one heavy chain and one light chain. Antibody heavy chains can form dimers, thus the heavy chain of one half-body can associate with heavy chain associated with a different molecule (*e.g.*, another half-body) or another Fc-containing polypeptide. Two slightly different Fc-domains may "heterodimerize" as in the formation of bispecific antibodies or other heterodimers, -trimers, -tetramers, and the like. See Vincent and Murini, "Current strategies in antibody engineering: Fc engineering and pH-dependent antigen binding, bispecific antibodies and antibody drug conjugates," 7 Biotechnol. J. 1444-1450 (20912); and Shimamoto et al., "Peptibodies: A flexible alternative format to antibodies," 4(5) MAbs 586-91 (2012).

In one aspect, the half-body variable domain specifically recognizes the internalization effector and the half body Fc-domain dimerizes with an Fc-fusion protein that comprises a replacement enzyme (*e.g.,* a peptibody) Id, 586.

"Alpha-glucosidase" (or "α-glucosidase"), "α-glucosidase activity", "GAA", and "GAA activity" are used interchangeably and refer to any protein that facilitates the hydrolysis of 1,4-alpha bonds of glycogen and starch into glucose. GAA is also known *inter alia* as EC 3.2.1.20, maltase, glucoinvertase, glucosidosucrase, maltase-glucoamylase, alpha-glucopyranosidase, glucosidoinvertase, alpha-D-glucosidase, alpha-glucoside hydrolase, alpha-1,4-glucosidase, and alpha-D-glucoside glucohydrolase. GAA can be found in the lysosome and at the brush border of the small intestine. Patients who suffer from Pompe disease lack functioning lysosomal α-glucosidase. See S. Chiba, "Molecular mechanism in alpha-glucosidase and glucoamylase," 61(8) Biosci. Biotechnol. Biochem. 1233-9 (1997); and Hesselink et al., "Lysosomal dysfunction in muscle with special reference to glycogen storage disease type II," 1637(2) Biochim. Biophys. Acta. 164-70 (2003).

"Alpha-galactosidase A "(or "α-galactosidase A"), "α-galactosidase A activity", "α-galactosidase", "α-galactosidase activity", "GLA", and "GLA activity" are used interchangeably and refer to any protein that facilitates the hydrolysis of terminal α-galactosyl moieties from glycolipids and glycoproteins, and also hydrolyses α-D-fucosides. GLA is also known *inter alia* as EC 3.2.1.22, melibiase, α-D-galactosidase, α-galactosidase A, α-galactoside galactohydrolase, α-D-galactoside galactohydrolase. GLA is a lysosomal enzyme encoded by the X-linked GLA gene. Defects in GLA can lead to Fabry Disease, in which the glycolipid known as globotriaosylceramide (a.k.a. Gb3, GL-3, or ceramide trihexoside) accumulates within blood vessels (*i.e.,* prominent vasculopathy), resulting in pain and impairment in the function of kidney, heart, skin, and/or cerebrovascular tissues. and other tissues, and organs. See for example Prabakaran et al. "Mannose 6-phosphate receptor and sortilin mediated endocytosis of α-galactosidase A in kidney endothelial cells," 7(6) PLoS One e39975 pp. 1-9 (2012).

In one aspect, the disclosure is a method of treating a patient (or subject) suffering from a lysosomal storage disease (LSD) by administering to the patient a "biotherapeutic complex". The biotherapeutic complex enters the cells of the patient and delivers to the lysosomes an enzyme or enzymatic activity that (*i.e.*, "replacement enzyme") that replaces the enzyme or enzymatic activity that is associated with the LSD (i.e, "endogenous enzyme").

In the present invention there is provided a biotherapeutic complex for use in a method of treating a subject suffering from a LSD, wherein the method comprises administering to the subject the biotherapeutic complex and wherein the biotherapeutic complex enters a lysosome of a cell of the subject and provides an enzyme activity ("replacement enzyme") that replaces the enzymatic activity that is associated with the LSD ("endogenous enzyme"), wherein the LSD is Pompe disease, and wherein the biotherapeutic complex comprises: (a) the replacement enzyme, wherein the replacement enzyme is α-glucosidase (GAA), and (b) an antigen binding protein that binds an internalization effector, wherein the antigen-binding protein is an antibody fragment covalently linked to the replacement enzyme, and wherein the internalization effector is transferrin receptor.

LSDs include sphingolipidoses, a mucopolysaccharidoses, and glycogen storage diseases. In some aspects, the LSD is any one or more of Fabry disease, Gaucher disease type I, Gaucher disease type II, Gaucher disease type III, Niemann-Pick disease type A, Niemann-Pick disease type BGM1-gangliosidosis, Sandhoff disease, Tay-Sachs disease, GM2- activator deficiency, GM3-gangliosidosis, metachromatic leukodystrophy, sphingolipid-activator deficiency, Scheie disease, Hurler-Sceie disease, Hurler disease, Hunter disease, Sanfilippo A, Sanfilippo B, Sanfilippo C, Sanfilippo D, Morquio syndrome A, Morquio syndrome B, Maroteaux-Lamy disease, Sly disease, MPS IX, and Pompe disease. In a specific aspect, the LSD is Fabry disease. In the biotherapeutic complex for use of the present invention the LSD is Pompe disease.

In some aspects, the biotherapeutic complex comprises (a) the replacement enzyme, and (b) a molecular entity that binds an internalization effector. In some cases, the replacement enzyme is any one or more of α-galactosidase, β-galactosidase, α-glucosidase, β-glucosidase, saposin-C activator, ceramidase, sphingomyelinase, β-hexosaminidase, GM2 activator, GM3 synthase, arylsulfatase, sphingolipid activator, α-iduronidase, iduronidase-2- sulfatase, heparin *N*-sulfatase, *N*-acetyl-α-glucosaminidase, α-glucosamide *N*-acetyltransferase, *N*-acetylglucosamine-6-sulfatase, *N*-acetylgalactosamine-6-sulfate sulfatase, *N*-acetylgalactosamine-4-sulfatase, β-glucuronidase, and hyaluronidase.

In some cases, the patient may not make sufficient protein such that a replacement enzyme is recognized by the patient as "non-self' and an immunological reaction ensues after administering a replacement enzyme. This is not desirable. Therefore, in some aspects, the replacement enzyme is designed or produced in such a way as to avoid inducing an immunological reaction in the subject. One such solution is to use an "isozyme" as a replacement enzyme. An isozyme is sufficiently close to a "self" protein of the patient, but has the replacement enzyme activity sufficient to ameliorate the symptoms of the LSD.

In one particular aspect, in which the LSD is Pompe disease and the endogenous enzyme is α-glucosidase (GAA), the isozyme can be any one of acid α-glucosidase, sucrase-isomaltase (SI), maltase-glucoamylase (MGAM), glucosidase II (GANAB), and neutral a-glucosidase (C GNAC). In another particular aspect, in which the LSD is Fabry disease and the endogenous enzyme is α-galactosidase A (GLA), the isozyme can be an α-*N*- acetylgalactosaminidase engineered to have GLA activity.

The biotherapeutic complex has an internalization effector binding protein component that enables the uptake of the replacement enzyme into the cell. Thus, in some aspects, the internalization effector can be CD63, MHC-I, Kremen-1, Kremen-2, LRP5, LRP6, LRP8, transferrin receptor, LDL-receptor, LDL-related protein 1 receptor, ASGR1, ASGR2, amyloid precursor protein-like protein-2 (APLP2), apelin receptor (APLNR), PRLR (prolactin receptor), MAL (Myelin And Lymphocyte protein, a.k.a. VIP17), IGF2R, vacuolar-type H+ ATPase, diphtheria toxin receptor, folate receptor, glutamate receptors, glutathione receptor, leptin receptor, scavenger receptor, SCARA1-5, SCARB1-3, and CD36. In certain aspects, the internalization effector is a kidney specific internalizer, such as CDH16 (Cadheri-16), CLDN16 (Claudn-16), KL (Klotho), PTH1R (parathyroid hormone receptor), SLC22A13 (Solute carrier family 22 member 13), SLC5A2 (Sodium/glucose cotransporter 2), and UMOD (Uromodulin). In other certain aspects, the internalization effector is a muscle specific internalizer, such as BMPR1A (Bone morphogenetic protein receptor 1A), m-cadherin, CD9, MuSK (muscle-specific kinase), LGR4/GPR48 (G protein-coupled receptor 48), cholinergic receptor (nicotinic) alpha 1, CDH15 (Cadheri-15), ITGA7 (Integrin alpha-7), CACNG1 (L-type calcium channel subunit gamma-1), CACNAlS (L-type calcium channel subunit alpha-15), CACNG6 (L-type calcium channel subunit gamma-6), SCN1B (Sodium channel subunit beta-1), CHRNA1 (ACh receptor subunit alpha), CHRND (ACh receptor subunit delta), LRRC14B (Leucine-rich repeat-containing protein 14B), and POPDC3 (Popeye domain-containing protein 3). In some specific aspects, the internalization effector is ITGA7, CD9, CD63, APLP2, or PRLR. In the present invention, the internalization effector is transferrin receptor.

In some aspects, the internalization effector-binding protein comprises an antigen-binding protein, which includes for example a receptor-fusion molecule, a trap molecule, a receptor-Fc fusion molecule, an antibody, an Fab fragment, an F(ab')2 fragment, an Fd fragment, an Fv fragment, a single-chain Fv (scFv) molecule, a dAb fragment, an isolated complementarity determining region (CDR), a CDR3 peptide, a constrained FR3-CDR3-FR4 peptide, a domain-specific antibody, a single domain antibody, a domain-deleted antibody, a chimeric antibody, a CDR-grafted antibody, a diabody, a triabody, a tetrabody, a minibody, a nanobody, a monovalent nanobody, a bivalent nanobody, a small modular immunopharmaceutical (SMIP), a camelid antibody (VHH heavy chain homodimeric antibody), and a shark variable IgNAR domain.

In one aspect, the molecular entity that binds the internalization effector is an antibody, an antibody fragment, or other antigen-binding protein. For example, the molecular entity can be a bispecific antibody, in which one arm binds the internalization effector (*e.g.,* ITGA7, CD9, CD63, PRLR, APLP2), and the other arm binds the replacement enzyme. Here, the biotherapeutic complex comprises the bispecific antibody and the replacement enzyme (Fig. 1A). In a specific aspect, the disease treated is Fabry disease, and the biotherapeutic complex comprises GLA and a bispecific antibody that binds GLA and CD63. In another specific aspect, the disease treated is Pompe disease, and the biotherapeutic complex comprises GAA and a bispecific antibody that binds GAA and CD63.

In another aspect, the molecular entity that binds the internalization effector comprises a half-antibody, and the replacement enzyme contains an Fc domain (enzyme-Fc fusion polypeptide). In one aspect, the Fc domain of the enzyme-Fc fusion polypeptide associates with the Fc domain of the internalization effector-specific half-body to form the biotherapeutic complex (Fig. 1B).

In other aspects, the replacement enzyme is covalently linked to internalization effector-binding protein. The enzyme-Fc fusion:half-body example described in the previous paragraph (see also Fig. 1B) falls into this class, since the Fc dimer can be secured via one or more disulfide bridges. The covalent linkage between the enzyme activity domain or polypeptide and the internalization-binding domain or polypeptide may be any type of covalent bond, *i.e.,* any bond that involved sharing of electrons. In some cases, the covalent bond is a peptide bond between two amino acids, such that the replacement enzyme and the internalization effector-binding protein in whole or in part form a continuous polypeptide chain, as in a fusion protein. In some cases, the replacement enzyme portion and the internalization effector-binding protein are directly linked. In other cases, a linker is used to tether the two portions. See Chen et al., "Fusion protein linkers: property, design and functionality," 65(10) Adv Drug Deliv Rev. 1357-69 (2013).

In a particular aspect, the replacement enzyme is covalently linked to the C- terminus of the heavy chain of an anti-internalization effector antibody (see Fig. 1C) or to the C-terminus of the light chain (Fig. 1E). In another particular aspect, the replacement enzyme is covalently linked to the N-terminus of the heavy chain of an anti-internalization effector antibody (see Fig. 1D) or to the N-terminus of the light chain (Fig. 1F).

In some cases, especially where the replacement enzyme is not normally proteolytically processed in the lysosome, a cleavable linker is added to those aspects of the biotherapeutic complex that comprise an antibody-enzyme fusion. In some aspects, a cathepsin cleavable linker is inserted between the antibody and the replacement enzyme to facilitate removal of the antibody in the lysosome in order to a) possibly help preserve enzymatic activity by removing the sterically large antibody and b) possibly increase lysosomal half-life of the enzyme.

In another aspect, the disclosure is a composition comprising an enzyme activity and an antigen-binding protein, wherein the enzyme is associated with a lysosomal storage disease (LSD) and internalization effector-binding protein. Enzymes (which include proteins that are not per se catalytic) associated with lysosomal storage diseases include for example α-galactosidase, β-galactosidase, α-glucosidase, β-glucosidase, saposin-C activator, ceramidase, sphingomyelinase, β-hexosaminidase, GM2 activator, GM3 synthase, arylsulfatase, sphingolipid activator, α-iduronidase, iduronidase-2-sulfatase, heparin *N*-sulfatase, *N*-acetyl-α-glucosaminidase, α-glucosamide *N*-acetyltransferase, *N*-acetylglucosamine-6-sulfatase, *N*-acetylgalactosamine-6-sulfate sulfatase, *N*-acetylgalactosamine-4-sulfatase, β-glucuronidase, hyaluronidase, and the like. In the present invention, there is provided a composition comprising an enzyme associated with a lysosomal storage disease (LSD) and an antigen-binding protein, wherein the enzyme is α-glucosidase (GAA), wherein the antigen-binding protein is an antibody fragment covalently linked to the enzyme, wherein the antigen-binding protein binds an internalization effector, and wherein the internalization effector is transferrin receptor.

Internalization effector-binding proteins for example include a receptor-fusion molecule, a trap molecule, a receptor-Fc fusion molecule, an antibody, an Fab fragment, an F(ab')2 fragment, an Fd fragment, an Fv fragment, a single-chain Fv (scFv) molecule, a dAb fragment, an isolated complementarity determining region (CDR), a CDR3 peptide, a constrained FR3-CDR3-FR4 peptide, a domain-specific antibody, a single domain antibody, a domain-deleted antibody, a chimeric antibody, a CDR-grafted antibody, a diabody, a triabody, a tetrabody, a minibody, a nanobody, a monovalent nanobody, a bivalent nanobody, a small modular immunopharmaceutical (SMIP), a camelid antibody (VHH heavy chain homodimeric antibody), a shark variable IgNAR domain, other antigen-binding proteins, and the like.

Internalization effectors include for example CD63, MHC-I, Kremen-1, Kremen- 2, LRP5, LRP6, LRP8, transferrin receptor, LDL-receptor, LDL-related protein 1 receptor, ASGR1, ASGR2, amyloid precursor protein-like protein-2 (APLP2), apelin receptor (APLNR), PRLR (prolactin receptor), MAL (Myelin And Lymphocyte protein, a.k.a. VIP17), IGF2R, vacuolar-type H+ ATPase, diphtheria toxin receptor, folate receptor, glutamate receptors, glutathione receptor, leptin receptor, scavenger receptor, SCARA1-5, SCARB1-3, and CD36. In certain aspects, the internalization effector is a kidney specific internalizer, such as CDH16 (Cadheri-16), CLDN16 (Claudn-16), KL (Klotho), PTH1R (parathyroid hormone receptor), SLC22A13 (Solute carrier family 22 member 13), SLC5A2 (Sodium/glucose cotransporter 2), and UMOD (Uromodulin). In other certain aspects, the internalization effector is a muscle specific internalizer, such as BMPR1A (Bone morphogenetic protein receptor 1A), m-cadherin, CD9, MuSK (muscle-specific kinase), LGR4/GPR48 (G protein-coupled receptor 48), cholinergic receptor (nicotinic) alpha 1, CDH15 (Cadheri-15), ITGA7 (Integrin alpha-7), CACNG1 (L-type calcium channel subunit gamma-1), CACNAlS (L-type calcium channel subunit alpha-15), CACNG6 (L-type calcium channel subunit gamma-6), SCN1B (Sodium channel subunit beta-1), CHRNA1 (ACh receptor subunit alpha), CHRND (ACh receptor subunit delta), LRRC14B (Leucine-rich repeat-containing protein 14B), and POPDC3 (Popeye domain-containing protein 3),In some specific aspects, the internalization effector is ITGA7, CD9, CD63, ALPL2, or PPRLR. In the compositions of the present invention the internalization effector is transferrin receptor

In some aspects, the enzyme is covalently linked (*i.e.,* electrons shared across atoms) to the antigen-binding protein. In one particular aspect, the internalization effector-binding protein consists of or contains a half-body; the enzyme is fused to an Fc-fusion domain (*e.g.,* at the C-terminus); and the Fc-domain that is covalently linked to the enzyme associates with the Fc-domain of the antigen-binding protein, such that the association contains one or more disulfide bridges. This particular aspect is schematically depicted in Figure 1B.

In another particular aspect, the internalization effector-binding protein (IE- BP) consists of or contains an antibody or an antibody fragment, and the enzyme is covalently linked to the antibody or antibody fragment. In a specific aspect, the IEBP is an antibody, and the enzyme is covalently linked (directly through a peptide bond, or indirectly via a linker) to the C-terminus of the heavy chain or the light chain of the antibody (Figure 1C or 1E, respectively). In another specific aspect, the IEBP is an antibody, and the enzyme is covalently linked (directly through a peptide bond, or indirectly via a linker) to the N-terminus of the heavy chain or the light chain of the antibody (Figure 1D or 1F, respectively).

In some aspects, the enzyme and IEBP are not covalently linked, but are combined in an admixture. The IEBP and the enzyme can associate through non-covalent forces to form a complex. For example, in one particular aspect, the IEBP is a bispecific antibody in which one arm of the antibody binds the internalization effector and the other arm binds the enzyme. This aspect is schematically depicted in Figure 1A.

In some aspects, the enzyme is GAA or comprises GAA activity (e.g., an isozyme with GAA activity), and the internalization effector is ITGA7, CDH15, CD9, CD63, APLP2, or PRLR. In a particular aspect, the enzyme is GAA or comprises GAA activity, the internalization domain is CD63, and the IEBP is a bispecific antibody with specificity for CD63 and GAA.

In some aspects, the enzyme is GLA or comprises GLA activity (*e.g.,* an isozyme with GAA activity), and the internalization effector is ITGA7, CD9, CD63, APLP2, or PRLR. In a particular aspect, the enzyme is GLA or comprises GLA activity, the internalization domain is CD63, and the IEBP is a bispecific antibody with specificity for CD63 and GLA.

In another aspect, the disclosure is a method for selecting or screening a biotherapeutic complex containing an enzyme and an antigen-binding protein that effectively replaces an enzyme in a patient in need thereof. In one aspect, the biotherapeutic complex is administered to a model system and the model system is assessed for replaced enzyme activity. In one aspect, the model system is an animal that lacks expression of the enzyme and expresses an antigen cognate of the antigen-binding protein. In one aspect, the animal model is a mouse that expresses a humanized cognate of the antigen-binding protein and with a knock-out of the gene that encodes the enzyme.

In one aspect, the mouse contains a knock-out of a lysosomal enzyme such as α-Galactosidase A, Ceramidase, β-Glucosidase, Saposin-C activator, Sphingomyelinase, β - Galactosidase, β -Hexosaminidase A and B, β -Hexosaminidase A, GM2-activator protein, GM3 synthase, Arylsulfatase A, Sphingolipid activator, α-Iduronidase, Iduronidase-2-sulphatase, Heparan N-sulphatase, N-acetyl-α-glucosaminidase, Acetyl-CoA; α-glucosamide N-acetyltransferase, N-acetylglucosamine-6- sulphatase, N-acetylgalactosamine-6- sulphate sulphatase, β -Galactosidase, N-acetylgalactosamine-4- sulphatase (arylsulphatase B), β - Glucuronidase, Hyaluronidase, α-Glucosidase 2 or Lysosomal acid lipase. In one aspect, the knock-out mouse also expresses the human or humanized version of the internalization effector (*i.e.,* cognate of the antigen-binding protein). Human internalization effectors include CD63, MHC-I, Kremen-1, Kremen-2, LRP5, LRP6, LRP8, transferrin receptor, LDL-receptor, LDL-related protein 1 receptor, ASGR1, ASGR2, amyloid precursor protein-like protein-2 (APLP2), apelin receptor (APLNR), PRLR (prolactin receptor), MAL (Myelin And Lymphocyte protein, a.k.a. VIP17), IGF2R, vacuolar-type H+ ATPase, diphtheria toxin receptor, folate receptor, glutamate receptors, glutathione receptor, leptin receptor, scavenger receptor, SCARA1-5, SCARB1-3, CD36, CDH16 (Cadheri-16), CLDN16 (Claudn-16), KL (Klotho), PTH1R (parathyroid hormone receptor), SLC22A13 (Solute carrier family 22 member 13), SLC5A2 (Sodium/glucose cotransporter 2), UMOD (Uromodulin), BMPR1A (Bone morphogenetic protein receptor 1A), M-cadherin, CD9, MuSK (muscle-specific kinase), LGR4/GPR48 (G protein-coupled receptor 48), cholinergic receptor (nicotinic) alpha 1, CDH15 (Cadheri-15), ITGA7 (Integrin alpha-7), CACNG1 (L-type calcium channel subunit gamma-1), CACNAlS (L-type calcium channel subunit alpha-15), CACNG6 (L-type calcium channel subunit gamma-6), SCN1B (Sodium channel subunit beta-1), CHRNA1 (ACh receptor subunit alpha), CHRND (ACh receptor subunit delta), LRRC14B (Leucine-rich repeat-containing protein 14B), and POPDC3 (Popeye domain-containing protein 3).

Methods of making mice that express human receptors are known in the art. See for example Ma et al., Drug Metab. Dispos. 2008 Dec; 36(12):2506-12; and U.S. Pat. No. 8,878,001 B2. Methods of making enzyme knock-out mice are also known in the art. See for example Kuemmel et al., Pathol. Res. Pract. 1997;193(10):663-71.

The following examples are provided to further illustrate the methods described herein. These examples are illustrative only and are not intended to limit the scope of the invention in any way.

### EXAMPLES

### Example 1: Lysosomal α-Glucosidase Fusion Protein

CI-MPR independent antibody-guided delivery systems were designed to deliver enzymes to the lysosome. Table 3 lists the molecular constructs, along with their levels of expression in CHO cells (see Figure 2), approximate GAA activity as determined using the fluorescent substrate 4-methylumbelliferyl-α-glucoside (see Figure 3), and lysosomal targeting, internalization, and activity status (see Figures 4 and 5).

### Example 2: GAA Construct Internalization

The internalization of GAA constructs was determined by measuring enzyme activity in cell lysates. Various constructs of recombinant GAA (SEQ ID NO: 1) were added to HEK293, human skeletal myoblasts (Lonza, Walkersville, MD), or C2C12 mouse myoblasts. Cells were plated in 24-well plates 24 hours prior to addition of enzyme constructs. Enzyme constructs were added to the media of cells for 18 hours. Cells were then extensively washed in ice-cold PBS and lysed in ice-cold 0.5% NP-40 in assay buffer (0.2 M sodium acetate, 0.4 M potassium chloride, pH 4.3). Lysates were centrifuged at 15,000 x g for 15 minutes at 4°C and supernatants were incubated with 4-methylumbelliferyl alpha-D-glucoside, a GAA substrate, in assay buffer for 1 hour in 96 well plates. The reactions were stopped using a glycine-carbonate buffer at pH 10.7. Fluorescence was read on a plate reader at 360nm excitation and 450nm emission. Lysate protein concentration was assayed using a bicinchoninic acid assay kit. 4-methylumbelliferone was used as standards. GAA activity was reported as nmol 4-methylumbelliferone released per hour per mg of purified protein. See Fuller et al., "Isolation and characterisation of a recombinant, precursor form of lysosomal acid alpha-glucosidase," 234(3) European Journal of Biochemistry 903-909 (1995).

Internalization of GAA activity was assessed in the presence or absence of mannose 6-phosphate (M6P) (Fig. 4A), and in the presence or absence of CD63. Some lysosomal enzymes are targeted to the lysosome via an attached M6P moiety binding to a mannose 6-phosphate receptor (MPR). Therefore, the involvement of CI-MPR on internalization of anti-CD63-GAA was assessed by including 5 mM M6P, which competes for MPR binding, in the HEK culture media during anti-CD63-GAA uptake. The inclusion of M6P had no effect on the uptake of anti-CD63-GAA as determined by the detection of 4-methylumbelliferone in cell lysates (Figure 4A). However, the uptake of anti-CD63-GAA was dependent upon the presence of CD63 (Figure 4B). Anti- CD63 • GAA was taken-up by HEK cells that express CD63, but was not taken-up by HEK cells carrying a knock-out of the CD63 gene (Figure 4B).

**Table 3**

| **Construct** | **CHO expression** | **GAA activity (*in vitro*)** | **Targeting** | | | |
|---|---|---|---|---|---|---|
| | | | **GAA activity** | **MPR-dependent** | **CD63-dependent** | **Lysosomal colocalization** |
| Anti-CD63 IgG4 | ++ | NA | No | No | Yes | Yes |
| GAA-Fc fusion (see Fig. 1B(i)) | + | + | NA | NA | NA | NA |
| GAA-Fc•anti-CD63 (see Fig. 1B) | NA | + | Yes | No | Yes | NT |
| Anti-CD63-GAA (see Fig. 1C) | ++ | + | Yes | No | Yes | Yes |
| GAA-anti-CD63 (see Fig. 1D) | + | + | Yes | No | Yes | NT |
| GAA | NT | + | Yes | Yes | No | NT |
| GLA | NA | - | NA | Yes | No | NT |
| •GLA• Anti-CD63 | NA | - | NA | No | Yes | NT |

| | | | | | | |
|---|---|---|---|---|---|---|
| NA = Not applicable; NT = Not tested | | | | | | |

### Example 3: Uptake of GAA Construct by Myoblasts

The ability of skeletal myoblasts, which is a tissue type of interest in Pompe disease, to take up various GAA constructs was tested. Human skeletal myoblasts were cultured in the presence of various concentrations (*i.e.,* 25 nM, 50 nM, and 200 nM) of (1) anti-CD63- GAA, (2) anti-CD63-GAA plus 5 mM M6P, (3) myc-GAA, and (4) myc-GAA plus 5 mM M6P. Internalization of the GAA constructs was inferred from the GAA activity level detected in cell lysates, as indicated by the accumulation of 4-methylumbelliferone, was assessed. Uptake of 200 nM anti-CD63-GAA, which was MPR-independent, was more than five-fold greater than the uptake of myc-GAA, which was dependent on MPR (Figure 5A).

To determine the subcellular localization of the anti-CD63-GAA construct, human skeletal myoblasts were stained with anti-human IgG antibodies (to detect the anti-CD63 moiety of the anti-CD63-GAA construct) and anti-LAMP antibodies (to label lysosomes). LAMP, or Lysosome-associated membrane glycoprotein, includes integral membrane proteins that are specific to lysosomes. The co-labeling experiment demonstrated that the anti-CD63-GAA co-localized with anti-LAMP, indicating that anti-CD63-GAA localizes to lysosomes.

Similarly, mouse C2C12 myoblasts were contacted with the various concentrations, *i.e.,* 25 nM, 50 nM, and 200 nM, of (1) anti-CD63-GAA, (2) anti-CD63-GAA plus 5 mM M6P, (3) myc-GAA, and (4) myc-GAA plus 5 mM M6P. Internalization of the GAA constructs was inferred from the GAA activity level detected in cell lysates, as indicated by the accumulation of 4-methylumbelliferone, was assessed. Uptake of 200 nM anti-CD63-GAA, which was not adversely affected by M6P, was more than about six-fold to about nine-fold greater than the uptake of myc-GAA (Figure 5B).

### Example 4: The Effect of Anti-CD63-GAA on Glycogen Accumulation

The effect of anti-CD63-GAA on glycogen accumulation in the lysosomes of three different Pompe cell lines was assessed. The Pompe cell lines were derived from fibroblasts obtained from severe onset infantile Pompe disease sufferers and contained knock-out or knock-down mutations in the GAA gene. The cells line used were GM20089 (exon 18 deletion), GM20090 (compound heterozygote in exon 14 and 16), and GM20090 (compound heterozygote in exon 2). These lines were obtained from Coriell Institute, Camden, NJ. See Huie et al., "Increased occurrence of cleft lip in glycogen storage disease type II (GSDII): exclusion of a contiguous gene syndrome in two patients by presence of intragenic mutations including a novel nonsense mutation Gln58Stop," 85(1) Am. J. Med. Genet. 5-8 (1999); Huie et al., "Glycogen storage disease type II: identification of four novel missense mutations (D645N, G648S, R672W, R672Q) and two insertions/deletions in the acid alpha-glucosidase locus of patients of differing phenotype," 244(3) Biochem. Biophys. Res. Commun. 921-7 (1998) and; Nishiyama et al., "Akt inactivation induces endoplasmic reticulum stress-independent autophagy in fibroblasts from patients with Pompe disease," 107 Molecular genetics and metabolism 490-5 (2012). Neonatal human dermal fibroblasts (NHDF, Lonza) were used as a control. All of the Pompe cell lines showed lysosomal glycogen accumulation (after 72 hours of glucose starvation to reduce cytoplasmic glycogen) even though residual (< 0.1%) GAA activity was present (Figure 6, panels A and B, respectively). Glycogen was measured using a glycogen assay kit (Sigma-Aldrich, St. Louis, MO).

Cells were glucose starved for about 96 hours prior to lysis to remove cytoplasmic glycogen (see Umapathysivam et al., "Correlation of acid alpha-glucosidase and glycogen content in skin fibroblasts with age of onset in Pompe disease," 361(1-2) Clin Chim Acta. 191-8 (2005)). Cells were treated with anti-CD63-GAA (200 nM) or myc-GAA (200 nM) for 72 hours prior to lysis. For all three Pompe cells lines, treatment with anti-CD63-GAA resulted in significant reduction in lysosomal glycogen accumulation (Figure 7), indicating that exogenously dosed anti-CD63-GAA reached the lysosome and was enzymatically active.

### Example 5: Isozymes to GAA

To avoid possible immune responses in Pombe patients to GAA (*i.e.,* cross- reactive immunological material or CRIM), the delivery of other human glucosidases to rescue GAA activity was investigated. Non-lysosomal enzymes with similar glycogen hydrolysis activity to GAA (a.k.a. acid α-glucosidase) are being investigated. Those enzymes include sucrase-isomaltase (SI), maltase-glucoamylase (MGAM), glucosidase II (GANAB), and neutral a-glucosidase (C GNAC). These enzymes and various recombinant embodiments are described in Dhital et al., "Mammalian mucosal α-glucosidases coordinate with α-amylase in the initial starch hydrolysis stage to have a role in starch digestion beyond glucogenesis," 8(4) PLoS One e625462013 Apr 25 (2013); Sim et al., "Human intestinal maltase-glucoamylase: crystal structure of the N-terminal catalytic subunit and basis of inhibition and substrate specificity," 375(3) J. Mol. Biol. 782-92 (2008); and Quezada-Calvillo et al., "Luminal starch substrate "brake" on maltase-glucoamylase activity is located within the glucoamylase subunit," 138(4) J. Nutr. 685-92 (2008).

The following isozyme constructs were made and expressed in CHO cells: (1) anti-CD63, (2) anti-CD63-NtMGAM (*i.e.,* the N-terminal subunit of maltase-glucoamylase linked to the C-terminus of the anti-CD63 antibody heavy chain), and (3) anti-CD63-CtMGAM. See Dhital, 2013. Another construct expressing an anti-mouse CD63 heavy chain fused to a C-terminal subunit of mouse maltase-glucoamylase (anti-mCD63-mctMGAM) was also engineered for use in mouse models (see Example 11).

### Example 6: Lysosomal α-Galactosidase A (GLA) Fusion Protein

Various fusion proteins containing human GLA (SEQ ID NO:2) were constructed and expressed in CHO cells. Those constructs included (i) GLA fused to the C-terminus of anti-CD63 heavy chains (anti-CD63-GLA), (ii) GLA fused to an immunoglobulin Fc (*e.g.,* Fc "knob"), (iii) GLA-anti-CD63 (*i.e.*, GLA fused to the N-terminus of anti-CD63 heavy chains), and (iv) GLA-myc fusion (Figure 8). Internalization effector binding proteins (IEBP) sans a GLA moiety were also engineered and expressed. In one case, the IEBP was a bispecific antibody with one half having binding specificity to CD63 and the other half having binding specificity to myc. GLA enzymatic activity (*i.e.,* hydrolysis of 4-methylumbeliferyl-β-galactopyranoside) was assessed for each GLA fusion protein, the results of which are presented in Figure 9. With the exception of the C-terminal anti-CD63-GLA fusion, all constructs showed α-galactosidase activity (Fig. 9). Fc knobs are described in Ridgway et al., "'Knobs-into-holes' engineering of antibody CH3 domains for heavy chain heterodimerization," 9(7) Protein Eng. 617-621 (1996).

The internalization of each construct (or combination of IEBP and GLA-myc) into HEK cells was determined by measuring the formation of methylumbelliferone from the enzyme catalyzed hydrolysis of 4-methylumbeliferyl-β-galactopyranoside. The various constructs were added to HEK293 cells, which were then incubated to allow endocytosis, then washed and lysed at pH 4. GLA substrate was added to each cell lysate and the α-galactosidase reaction was allowed to proceed. The results are shown in Figure 9, which shows that the GLA-anti-CD63 fusion protein was internalized into HEK cells. GLA-myc alone, *i.e.,* in the absence of an IEBP element, was not taken up by HEK cells. It was however taken up in the presence of a bispecific antibody that bound CD63 and bound the myc epitope (Figure 10).

### Example 7: Uptake of Internalization Effector-Binding Proteins

The internalization of anti-CD63 or anti-APLP2 antibodies into low pH cellular fractions was compared to the internalization of recombinant human GLA (rhGLA), which has high M6P content, into low pH fractions. Each of GLA, anti-CD63, and anti-APLP2 was labeled with the low pH sensitive dye pHrodo^{®} (Invitrogen, Calsbad, CA). HEK cells, HepG2 cells (liver carcinoma), and PC-3 cells (prostate cancer) were contacted with the pHrodo^{®}-labeled proteins and incubated overnight or for about 16 hours. The cells were then imaged and fluorescent vesicles were counted. Fluorescence output was normalized according to the degree of labeling for each protein. HEK, PC-3 and HepG2 cells demonstrated higher uptake of anti-CD63 and anti-APLP2 versus rhGLA (Figure 11).

### Example 8: Processing of fusion protein in lysosome

Whether the anti-CD63-GAA construct is proteolytically processed in the lysosome was tested by western blot analysis of Pompe cell lysates. GM20089 Pompe cells were cultured in the presence of anti-CD63-GAA and subsequent cell lysates were subjected to reduced western blot analysis using anti-GAA antibodies (Figure 12, panel A) or anti-hIgG antibodies (Figure 12, panel B).

The GM20089 Pompe disease fibroblast line from Coriell was plated into 6 well plates and incubated with anti-CD63-GAA for 18 hours and then extensively washed with media. Wells were lysed in RIPA buffer at various timepoints, *i.e.,* 0, 24, 48, 72 hours after removal of anti-CD63-GAA. Lysates were assayed for GAA by western blot using an anti-human GAA antibody (ab113021, Abcam LTD, Cambridge, UK; Figure 12). The full anti-CD63-GAA and smaller intermediates were detected in early timepoints, and the mature lysosomal 76kDa form of GAA was detected in all timepoints after dosing with anti-CD63-GAA. This demonstrates that anti-CD63-GAA internalizes in patient cells and is correctly processed to the mature lysosomal form of GAA. Furthermore, the half-life of the 76kDa form in the cells matches other studies of GAA internalization (Maga, J. A. et al. Glycosylation-independent lysosomal targeting of acid α-glucosidase enhances muscle glycogen clearance in Pompe mice. Journal of Biological Chemistry 288, 1428-1438 (2013).)

### Example 9: Tissue distribution and processing of anti-CD63-GAA

Mice humanized at the CD63 locus (see Valenzuela et al., "High-throughput engineering of the mouse genome coupled with high-resolution expression analysis," 21 Nature Biotechnology 652 - 659 (2003)) were administered anti-CD63-GAA. Tissue samples were taken and GAA was assessed via western blot analysis. GAA was detected in liver, diaphragm, kidney, heart, and quadriceps and gastrocnemius muscles (Figure 13).

Briefly, humanized CD63 mice were created by knocking the human CD63 locus into the mouse CD63 locus. Anti-CD63-GAA or anti-CD63 was administered by tail-vein injection at 50mg/kg to 2 month old humanized CD63 mice or wild-type mice lacking human CD63. Tail bleeds were performed at various time points, *e.g.,* 0, 6, 24 hours after injection. An anti-human Fc ELISA was performed to determine serum concentration of anti-CD63-GAA. Anti-CD63-GAA was cleared rapidly from serum and was faster than the parental anti-CD63 antibody. Injected mice were also sacrificed at various time points and tissues were dissected and snap frozen in liquid nitrogen. Tissues were lysed in RIPA buffer and assayed for GAA by western blot. The 76kDa mature lysosomal form was present in most tissues assayed, demonstrating internalization of anti-CD63-GAA into cells within tissue (Figure 13). Humanized CD63 mice internalized anti-CD63-GAA in skeletal muscle (heart, gastrocnemius, quadriceps), but wild-type mice did not show any uptake. This demonstrates that anti-CD63-GAA internalized into skeletal muscle cells mediated by CD63.

CD63 humanized mice (CD63^{hu/hu}) and control mice (CD63^{+/+} ) were administered anti-CD63-GAA at 50 mg/kg via tail vein injection. At the 24 hour time point, tissues were extracted and 200 µg of lysate was loaded per lane. The western blot was probed with anti-hGAA antibody and anti-GAPDH as a loading control. Figure 14 depicts the western blot and demonstrates that CD63-mediated the uptake of anti-hCD63-GAA into muscle tissue in CD63^{hu/hu} but not in WT CD63^{+/+} mice. The anti-hCD63-GAA was processed into mature 76 kDa hGAA.

### Example 10: Muscle-specific internalization effectors

Biotinylated antibodies to muscle-specific antigens, which included anti-integrin alpha 7, anti-CD9, and anti-dystroglycan, were administered to GAA knock-out mice. The tissue distributions of those antibodies were determined by western blot analysis. Figure 15 depicts a histogram of the level of antibody found within skeletal muscle (gastrocnemius, quadriceps, and diaphragm), heart muscle, liver (serving as the baseline for normalization), kidney and spleen. Anti-integrin alpha 7 antibodies were found (in levels exceeding the levels found in liver) in skeletal muscle and heart. Anti-CD9 antibodies were found (in levels exceeding the levels found in liver) in skeletal muscle and heart, and in kidney and spleen as well (Figure 15).

To determine whether those select muscle-specific antibodies can target lysosomes, the antibodies were labeled with pHrodo-red and then incubated overnight at 10 µg/mL with murine C2C12 myoblasts. Vesicular fluorescence was quantified and normalized to the degree of labeling of the fluorophore on the antibody. The results are depicted in Figure 16, which shows anti-CD63, anti-dystroglycan, anti-M-cadherin, anti-CD9, and anti-integrin alpha 7 targeted to the low pH lysosomal fraction of the C2C12 myoblasts.

### Example 11: Restoration of Muscle Glycogen Levels

2-3 month old GAA knock-out (KO) mice with native mouse CD63 expression were subjected to hydrodynamic delivery (HDD) of plasmid constructs encoding full-length human α-glucosidase (hGAA), anti-mCD63-GAA (anti-mouse CD63) and its associated light chain, or anti-hCD63-GAA (anti-human CD63) and its associated light chain. All constructs used identical plasmid backbones consisting of ubiquitin promoter and SV40 polyA tails. Briefly, 40 µg of each plasmid (*i.e.* 40µg of heavy and light chains, or only hGAA) was diluted in 2-3mL of sterile saline and rapidly injected into the tail-vein of GAA KO mice. Tail bleeds every 3 days demonstrated serum levels of hGAA or antibody-GAA constructs for ~10 days. Mice were sacrificed 3 weeks post-HDD, and tissues were snap frozen in liquid nitrogen. Tissues were homogenized in distilled water, boiled, and spun down. Supernatants were assayed for glycogen using a fluorescent glycogen assay kit (MAK016, Sigma Aldrich, St. Louis, MO)

Glycogen levels in heart, diaphragm, and skeletal muscle including triceps, gastrocnemius, and quadriceps were determined in wild-type mice, GAA KO mice, and GAA KO mice treated with hydrodynamically delivered and expressed hGAA or anti-mCD63-GAA. The results are depicted in Figure 17, which shows the restoration of glycogen to near wildtype levels in the anti-mCD63-GAA-treated mice. The glycogen restoration effect was more pronounced with anti-mCD63-GAA, which showed close to 100% restoration of wildtype muscle glycogen levels, than with hGAA alone, which showed only about 10% to 35% reduction in glycogen levels in skeletal muscle or diaphragm in GAA KO mice (see Fig. 17).

Glycogen levels in heart, diaphragm, and skeletal muscle including triceps, gastrocnemius, and quadriceps were determined in wild-type mice, GAA KO mice, and GAA KO mice treated with hydrodynamically delivered (HDD) and expressed anti-hCD63-GAA, anti-mCD63-GAA, or anti-mCD63-mctMGAM. The results depicted in Figure 18 show the restoration of glycogen to near wildtype levels in the anti-mCD63-GAA-treated mice. The glycogen restoration effect was more pronounced with anti-mCD63-GAA, which showed restoration of muscle glycogen levels to within 20% of wild-type levels, than with anti-hCD63-GAA alone, which showed only about 20% reduction in glycogen levels in skeletal muscle or diaphragm in GAA KO mice (see Fig. 18). This result further confirms the enhanced effect of GAA delivered to the lysosome via species-specific CD63 internalization. Treatment with anti-mCD63-mctMGAM via HDD in GAA KO mice compared to control (untreated) GAA KO mice were examined 7 or 12 days post HDD (see Fig. 20). Reduced glycogen was observed *in vivo* in the GAA KO mice treated with the mouse-specific anti-mCD63-mctMGAM HDD construct at both time points, while wild-type untreated mice otherwise under the same conditions show no changes in glycogen storage (Fig. 20).

### Example 12: Lysosomal Acid Lipase

Lysosomal acid lipase (LAL or LIPA) is an enzyme that breaks down cholesteryl esters and triglycerides in the lysosome. A deficiency in LIPA (*e.g.,* LAL-D or Wolman's disease) leads to a build-up of fatty material in the liver, spleen and other organs. The prevalence of LAL-D is between 1 in 40,000 and 1 in 300,000 people world-wide. Infants with LIPA deficiencies if untreated die within 6-12 months due to multi-organ failure. Older children may remain undiagnosed until they die from a heart attack, stroke or liver failure.

To test the effect of antibody tethering to LIPA on endogenous lipase activity, two LIPA antibody constructs were made: a heavy chain C-terminal fusion (anti-myc-LIPA); and a heavy chain N-terminal fusion (LIPA-anti-myc). The cDNA of amino acids 24-399 of the human lysosomal acid lipase enzyme (SEQ ID NO:3) was cloned into the N-terminus or C-terminus of an antibody heavy chain plasmid. A cathepsin cleavable sequence was used as a linker between the heavy chain and the enzyme. Constructs along with a corresponding light chain were transfected into CHO-K1 cells. CHO cell supernatants were collected 5 days after transfection and were sterile filtered. The supernatants were subjected to western blot analysis and probed with anti-LIPA antibody and anti-hIgG antibody. Expression of LIPA, anti-myc-LIPA, and LIPA-anti-myc in CHO cells were confirmed.

Dilutions of supernatants in assay buffer (0.2 M sodium acetate, 0.4 M potassium chloride, pH 4.3) were incubated with 4-methylumbelliferyl oleate, a LIPA substrate, for 1 hour. The reactions were stopped using a glycine-carbonate buffer at pH 10.7. Fluorescence was read on a plate reader at 360nm excitation and 450nm emission. Both the anti-myc-LIPA and LIPA-anti-myc constructs exhibited significant lipase activity relative to the native LIPA control (Figure 19).

## Claims

1. A composition comprising an enzyme associated with a lysosomal storage disease (LSD) and an antigen-binding protein,
wherein the enzyme is α-glucosidase (GAA),
wherein the antigen-binding protein is an antibody fragment covalently linked to the enzyme,
wherein the antigen-binding protein binds an internalization effector, and
wherein the internalization effector is transferrin receptor.

2. The composition of claim 1, wherein the enzyme comprises the amino acid sequence of SEQ ID NO: 1.

3. A biotherapeutic complex for use in a method of treating a subject suffering from a lysosomal storage disease (LSD), wherein the method comprises administering to the subject the biotherapeutic complex and wherein the biotherapeutic complex enters a lysosome of a cell of the subject and provides an enzyme activity ("replacement enzyme") that replaces the enzymatic activity that is associated with the LSD ("endogenous enzyme"),
wherein the LSD is Pompe disease, and
wherein the biotherapeutic complex comprises:
(a) the replacement enzyme, wherein the replacement enzyme is α-glucosidase (GAA), and
(b) an antigen binding protein that binds an internalization effector, wherein the antigen-binding protein is an antibody fragment covalently linked to the replacement enzyme, and wherein the internalization effector is transferrin receptor.

4. The biotherapeutic complex for use according to claim 3, wherein the replacement enzyme does not induce an immunological reaction in the subject.

5. The biotherapeutic complex for use according to claim 3 or 4, wherein the replacement enzyme comprises the amino acid sequence of SEQ ID NO: 1.

## Patentansprüche

1. Zusammensetzung, umfassend ein Enzym, das mit einer lysosomalen Speicherkrankheit (LSD) assoziiert ist, und ein antigenbindendes Protein,
wobei das Enzym α-Glucosidase (GAA) ist,
wobei das antigenbindende Protein ein Antikörperfragment ist, das kovalent mit dem Enzym verknüpft ist,
wobei das antigenbindende Protein einen Internalisierungseffektor bindet, und
wobei der Internalisierungseffektor ein Transferrinrezeptor ist.

2. Zusammensetzung nach Anspruch 1, wobei das Enzym die Aminosäuresequenz von SEQ ID NO: 1 umfasst.

3. Biotherapeutischer Komplex für die Verwendung in einem Verfahren zum Behandeln eines Subjekts, das an einer lysosomalen Speicherkrankheit (LSD) leidet, wobei das Verfahren das Verabreichen des biotherapeutischen Komplexes an das Subjekt umfasst und wobei der biotherapeutische Komplex in ein Lysosom einer Zelle des Subjekts eintritt und eine Enzymaktivität ("Ersatzenzym") bereitstellt, die die enzymatische Aktivität ersetzt, die mit der LSD assoziiert ist ("endogenes Enzym"),
wobei die LSD die Pompe-Krankheit ist, und
wobei der biotherapeutische Komplex umfasst:
(a) das Ersatzenzym, wobei das Ersatzenzym α-Glucosidase (GAA) ist, und
(b) ein antigenbindendes Protein, das einen Internalisierungseffektor bindet, wobei das antigenbindende Protein ein Antikörperfragment ist, das kovalent mit dem Ersatzenzym verknüpft ist und wobei der Internalisierungseffektor ein Transferrinrezeptor ist.

4. Biotherapeutischer Komplex für die Verwendung nach Anspruch 3, wobei das Ersatzenzym keine immunologische Reaktion in dem Subjekt auslöst.

5. Biotherapeutischer Komplex für die Verwendung nach Anspruch 3 oder 4, wobei das Ersatzenzym die Aminosäuresequenz von SEQ ID NO: 1 umfasst.

## Revendications

1. Composition comprenant un enzyme associé à une maladie de stockage lysosomal (MSL) et une protéine de liaison à l'antigène,
dans laquelle l'enzyme est α-glucosidase (GAA),
dans laquelle la protéine de liaison à l'antigène est un fragment d'anticorps relié de façon covalente à l'enzyme,
dans laquelle la protéine de liaison à l'antigène se lie à un effecteur d'internalisation, et
dans laquelle l'effecteur d'internalisation est le récepteur de transferrine.

2. Composition de la revendication 1, dans laquelle l'enzyme comprend la séquence d'acides aminés de SEQ ID n° : 1.

3. Complexe biothérapeutique pour utilisation dans une méthode de traitement d'un sujet souffrant d'une maladie de stockage lysosomal (MSL), dans lequel la méthode comprend l'administration, au sujet, du complexe biothérapeutique, et dans lequel le complexe biothérapeutique entre dans un lysosome d'une cellule du sujet et fournit une activité enzymatique (« enzyme de remplacement ») qui remplace l'activité enzymatique qui est associée à la MSL (« enzyme endogène »),
dans lequel la MSL est la maladie de Pompe, et
dans lequel le complexe biothérapeutique comprend :
(a) l'enzyme de remplacement, dans lequel l'enzyme de remplacement est α-glucosidase (GAA), et
(b) une protéine de liaison à l'antigène qui se lie à un effecteur d'internalisation, dans lequel la protéine de liaison à l'antigène est un fragment d'anticorps relié de façon covalente à l'enzyme de remplacement, et dans lequel l'effecteur d'internalisation est le récepteur de transferrine.

4. Complexe biothérapeutique pour utilisation selon la revendication 3, dans lequel l'enzyme de remplacement n'entraîne pas de réaction immunologique chez le sujet.

5. Complexe biothérapeutique pour utilisation selon la revendication 3 ou 4, dans lequel l'enzyme de remplacement comprend la séquence d'acides aminés de SEQ ID n° : 1.
